# EUROPEAN PATENT SPECIFICATION

(11) **EP 4 153 113 B1**
(45) Date of publication and mention of the grant of the patent: **11.11.2026**
(21) Application number: 21731661.1
(22) Date of filing: 19.05.2021
(51) Int. Cl.: A61F 13/49

(54) **ABSORBENT ARTICLES WITH WAISTBANDS AND WAISTBAND COVERS**
SAUGFÄHIGE ARTIKEL MIT TAILLENBÄNDERN UND TAILLENBANDABDECKUNGEN
ARTICLES ABSORBANTS MUNIS DE CEINTURES ET DE RECOUVREMENTS DE CEINTURES

(30) Priority: 22.05.2020 US 202063028614 P
(43) Date of publication of application: 29.03.2023
(73) Proprietor: The Procter & Gamble Company, Cincinnati, OH 45202 (US)
(72) Inventor: HEBBELER, Sarah, Ann, Cincinnati, Ohio 45202 (US); ROE, Donald, Carroll, Cincinnati, Ohio 45202 (US); MEIROSE, Aaron, Joseph, Cincinnati, Ohio 45202 (US)
(74) Representative: P&G Patent Germany
(86) International application number: PCT/US2021/033045
(87) International publication number: WO 2021/236700

(56) References cited:
- WO-A1-2009/084643
- WO-A1-92/22272
- US-A1- 2015 126 954
- US-A1- 2018 168 889

## Description

### FIELD

The present disclosure is generally directed to absorbent articles comprising waistbands and waistband covers.

### BACKGROUND

Absorbent articles such as diapers, training pants, and adult incontinence articles are used for receiving and containing urine, menses, and/or bowel movements (together "bodily exudates"). To effectively contain the bodily exudates, absorbent articles may provide a somewhat snug fit around a waist of a wearer. One way of providing a somewhat snug fit around the waist of the wearer may be the incorporation of a waistband into an absorbent article. Waistbands may be made with various stretch materials, for example elastic films or elastic strands. One method for creating a waistband may be the placement of a stretch material underneath a topsheet of the absorbent article to create an internal waistband. Another method for creating a waistband may be attachment of a stretch material on a wearer-facing or garment-facing surface of a chassis of the absorbent article, for example, on a wearer-facing surface of a topsheet or a garment-facing surface of an outer cover, to create an external waistband. The external waistband may also comprise one or more layers, such as a nonwoven layer, disposed between the stretch material and the skin of the wearer or the garment of the wearer.

Internal waistband applications may provide a more integrated and finished appearance as compared to external waistband applications. Internal waistbands, however, may also add increased complexity and cost to a manufacturing process. External waistbands, on the other hand, may be less complicated and less costly to apply to absorbent articles. However, external waistbands may be perceived as being less gentle to the skin of the wearer and may snag on garments of the wearer due to, for example, exposed edges and ridges of the waistband. For example, an external waistband disposed on a wearer-facing surface of an absorbent article may increase the incidence of red marking on the skin of a wearer, due to, for example, local pressure concentrations from exposed edges and ridges, which is not consumer desired. Exposed edges and ridges of an external waistband disposed on a garment-facing surface of an absorbent article may catch on the clothing of a wearer, causing the clothing to drape or wrinkle in an undesirable way. In addition, an external waistband disposed on the garment-facing surface of an absorbent article may provide a visually distracting break on the outer surface of the absorbent article, and may draw the attention of the wearer, who may pull at the edges, potentially partially or fully removing the waistband from the absorbent article.

WO200984643A1 relates to a pants type disposable diaper with a abdominal-side exterior sheet and a separated rear-side exterior sheet. The waist-side end portion and the waistline lower end portion of at least one of the abdominal-side exterior sheet and the rear-side exterior sheet are formed by laminating a plurality of layers of sheet-like breathable materials and an elastic stretchable member between the sheet-like breathable materials.

US20180168889A1 is concerned with absorbent article comprising an elastomeric laminate as part of a waistband, a waistcap, an inner leg cuff, an outer leg cuff or transverse barrier. The elastomeric laminate comprises one or more pluralities of elastics with defined properties, such as strand spacing and strain.

WO9222272A1 discloses absorbent articles with an elastic waist feature, a dual fastening system and elasticized side panels. The elasticized waistband, which is formed from a stretch laminate, comprises a panel that is capable of expanding well beyond the dimensions of the absorbent article set by the primary fastening system and the original dimensions. The extension forces of the elasticized waistband are lower than the extension forces of the elasticized side panels.

US20150126954A1 relates to disposable absorbent articles including a first waist region, a second waist region, at least one waistband in the first waist region and at least one fastener in the second waist region. The first waist region has a certain resiliency and stiffness.

As such, absorbent articles comprising external waistbands should be improved.

### SUMMARY

Aspects of the present disclosure solve one or more of the problems discussed above by providing an absorbent article with a waistband and a waistband cover. The waistband cover may reduce the amplitude of local pressure concentrations from, for example, exposed edges and ridges of the waistband. The waistband cover may also reduce the incidence and/or severity of red marking on the skin of the wearer due, in part, to increased local pressure concentrations. The waistband cover may also provide a more finished, integrated, and/or softer appearance to an absorbent article comprising an external waistband.

The invention provides an absorbent articles comprising a waistband cover as defined in the claims.

### Brief Description of the Drawings

The above-mentioned and other features and advantages of the present disclosure, and the manner of attaining them, will become more apparent and the disclosure itself will be better understood by reference to the following description of example forms of the disclosure taken in conjunction with the accompanying drawings, wherein:
Fig. 1 is a plan view of an example absorbent article in the form of a taped diaper, garment-facing surface facing the viewer, in a flat laid-out state;
Fig. 2 is a plan view of the example absorbent article of Fig. 1, wearer-facing surface facing the viewer, in a flat laid-out state;
Fig. 3 is a front perspective view of the absorbent article of Figs. 1 and 2 in a fastened position;
Fig. 4 is a front perspective view of an absorbent article in the form of a pant;
Fig. 5 is a plan view of the absorbent article of Fig. 4, laid flat, with a garment-facing surface facing the viewer;
Fig. 6 is a cross-sectional view of the absorbent article taken about line 6-6 of Fig. 5;
Fig. 7 is a cross-sectional view of the absorbent article taken about line 7-7 of Fig. 5;
Fig. 8 is a plan view of an example absorbent core or an absorbent article;
Fig. 9 is a cross-sectional view, taken about line 9-9, of the absorbent core of Fig. 8;
Fig. 10 is a cross-sectional view, taken about line 10-10, of the absorbent core of Fig. 8;
Fig. 11 is a plan view of a portion of the front waist region and a portion of the back waist region of an absorbent article of the present disclosure, wearer-facing surface facing the viewer, in a flat laid-out state;
Fig 12 is a plan view of a cut zone of a web of pre-cut absorbent articles of the present disclosure, showing a portion of the back waist region of one absorbent article and a portion of the front waist region of another absorbent article;
Figs. 13A-13E are schematic illustrations of various shapes of waistbands and waistband covers of the present disclosure;
Fig. 14 is a side view of a waistband laminate of the present disclosure;
Fig. 15 is a plan view of an absorbent article comprising a waistband and a waistband cover of the present disclosure, wearer-facing surface facing the viewer, in a flat laid-out state;
Fig. 16 is a plan view of a portion of the front waist region or the back waist region of an absorbent article comprising a waistband and a waistband cover of the present disclosure, wearer-facing surface facing the viewer, in a flat laid-out state;
Fig. 17 is a plan view of a portion of the front waist region or the back waist region of an absorbent article comprising a waistband and a waistband cover of the present disclosure, wearer-facing surface facing the viewer, in a flat laid-out state;
Fig. 18 is a plan view of a portion of the front waist region or the back waist region of an absorbent article comprising a waistband and a waistband cover of the present disclosure, wearer-facing surface facing the viewer, in a flat laid-out state;
Fig. 19 is a plan view of a waistband cover of the present disclosure;
Fig. 20 is a plan view of a portion of the front waist region or the back waist region of an absorbent article of the present disclosure comprising a waistband cover comprising bonds forming bond patterns, wearer-facing surface facing the viewer, in a flat laid-out state;
Fig. 21 is a schematic illustration of a patterned bond or aperture pattern, with bonds or apertures being black portions;
Fig. 22 is a schematic illustration of a bond or aperture pattern macro-array;
Figs. 23A-23C are schematic illustrations of waistband covers of the present disclosure comprising a pattern of color, a pattern of embossment, and/or printed indicia;
Fig. 24 is a cross-sectional view, taken about line 24-24, of the portion of the back waist region of the absorbent article of Fig. 20;
Fig. 25A is a cross-sectional view, taken about line 25-25, of the portion of the back waist region of the absorbent article of Fig. 20;
Fig. 25B is a cross-sectional view, taken about line 25-25, of the portion of the back waist region of the absorbent article of Fig. 20;
Fig. 26 is a plan view of a portion of the front waist region or the back waist region of an absorbent article of the present disclosure comprising a waistband partially free from overlap with a waistband cover, wearer-facing surface facing the viewer, in a flat laid-out state;
Fig. 27 is a plan view of a portion of the front waist region or back waist region of an absorbent article of the present disclosure comprising a stretch inhibition material, wearer-facing surface facing the viewer, in a flat laid-out state;
Fig. 28 is a cross-sectional view taken about line 28-28 of the portion of the front waist region or the back waist region of the absorbent article of Fig. 27;
Fig. 29 is a plan view of an absorbent article of the present disclosure comprising a waistband disposed on the garment-facing surface of the chassis, garment-facing surface facing the viewer, in a flat laid-out state; and
Fig. 30 is a plan view of a portion of the front waist region or the back waist region of an absorbent article comprising a waistband and a waistband cover of the present disclosure, wearer-facing surface facing the viewer, in a flat laid-out state.

### DETAILED DESCRIPTION

Various non-limiting forms of the present disclosure will now be described to provide an overall understanding of the principles of the structure, function, manufacture, and use of the absorbent articles with waistbands and waistband covers disclosed herein. One or more examples of these non-limiting forms are illustrated in the accompanying drawings. Those of ordinary skill in the art will understand that the absorbent articles with waistbands and waistband covers described herein and illustrated in the accompanying drawings are non-limiting example forms. The features illustrated or described in connection with one non-limiting form may be combined with the features of other non-limiting forms. Such modifications and variations are intended to be included within the scope of the present disclosure.

As used herein, the term "bond pattern" refers to a plurality of bonds having an overall design or pattern. The plurality of bonds may form a random, nonhomogeneous pattern, or may be grouped in arrays of bonds in which a pattern emerges in the plurality of bonds and/or in the unbonded space between the plurality of bonds. The bond arrays may comprise a plurality of bonds that are of generally consistent size and/or shape, or may comprise a plurality of bonds of varying sizes and/or shapes. The bond arrays may form a regular or recognizable shape, such as a heart shape, polygon, ellipse, arrow, chevron, sinusoidal wave, and/or other shapes known in the pattern art.

As used herein, the terms "hydrophilic" and "hydrophobic" have meanings that are well established in the art with respect to the contact angle of water on the surface of a material. Thus, a material having a water contact angle of greater than about 90 degrees is considered hydrophobic, and a material having a water contact angle of less than about 90 degrees is considered hydrophilic. Compositions which are hydrophobic may increase the contact angle of water on the surface of a material, while compositions which are hydrophilic may decrease the contact angle of water on the surface of a material. Notwithstanding the foregoing, reference to relative hydrophobicity or hydrophilicity between a material and a composition, between two materials, and/or between two compositions, does not imply that the materials or compositions are hydrophobic or hydrophilic. For example, a composition may be more hydrophobic than a material. In such a case, neither the composition nor the material may be hydrophobic; however, the contact angle exhibited by the composition is greater than that of the material. As another example, a composition may be more hydrophilic than a material. In such a case, neither the composition nor the material may be hydrophilic; however, the contact angle exhibited by the composition may be less than that exhibited by the material.

### General Description of an Absorbent Article

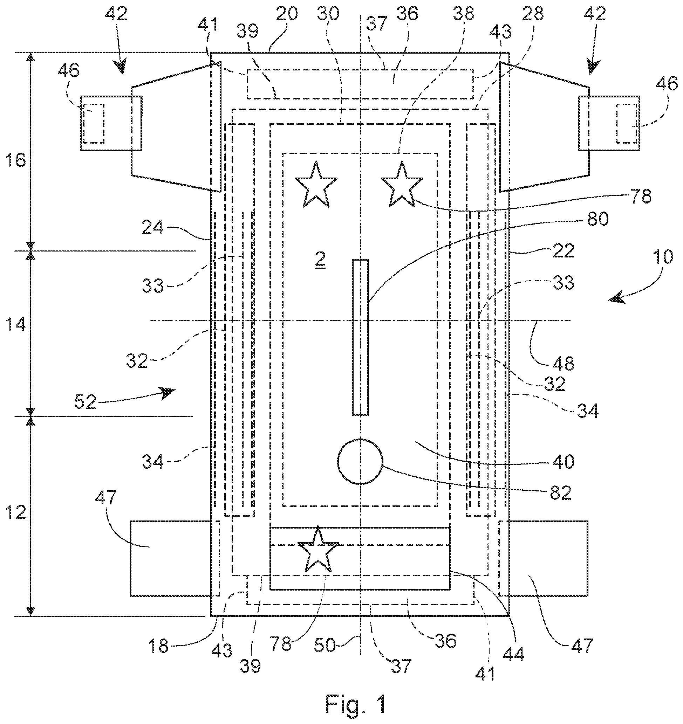

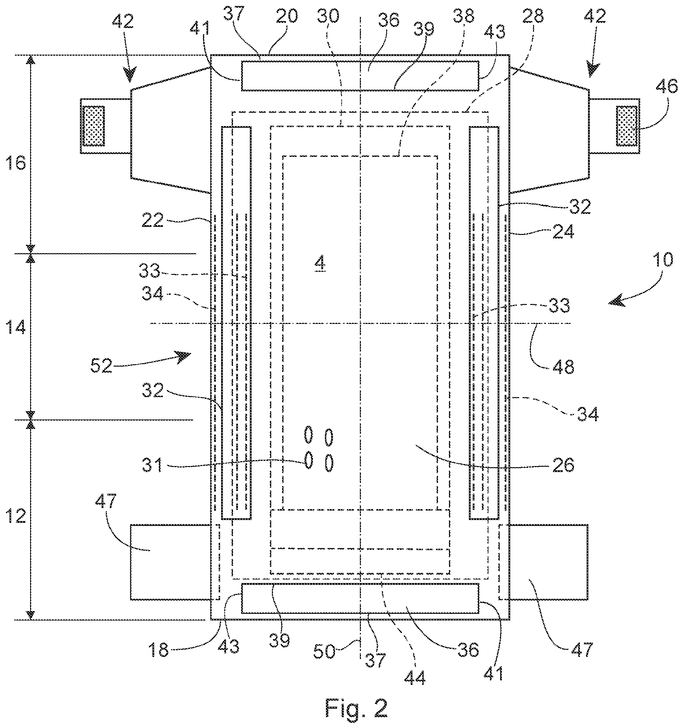

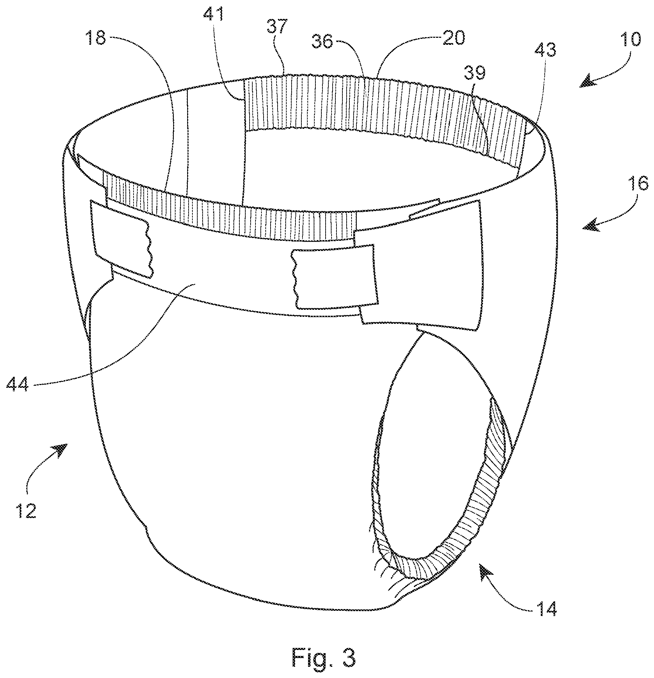

An example absorbent article 10 according to the present disclosure, shown in the form of a taped diaper, is represented in Figs. 1-3. Fig. 1 is a plan view of the example absorbent article 10, garment-facing surface 2 facing the viewer in a flat, laid-out state (i.e., no elastic contraction). Fig. 2 is a plan view of the example absorbent article 10 of Fig. 1, wearer-facing surface 4 facing the viewer in a flat, laid-out state. Fig. 3 is a front perspective view of the absorbent article 10 of Figs. 1 and 2 in a fastened configuration. The absorbent article 10 of Figs. 1-3 is shown for illustration purposes only as the present disclosure may be used for making a wide variety of diapers, including adult incontinence products, pants, or other absorbent articles, such as sanitary napkins and absorbent pads, for example.

The absorbent article 10 comprises a front waist region 12, a crotch region 14, and a back waist region 16. The crotch region 14 extends intermediate the front waist region 12 and the back waist region 16. The front waist region 12, the crotch region 14, and the back waist region 16 may each be 1/3 of the length of the absorbent article 10. The absorbent article 10 may comprise a front end edge 18, a back end edge 20 opposite to the front end edge 18, and longitudinally extending, transversely opposed side edges 22 and 24 defined by the chassis 52.

The absorbent article 10 comprises a liquid permeable topsheet 26, a liquid impermeable backsheet 28, and an absorbent core 30 positioned at least partially intermediate the topsheet 26 and the backsheet 28. The absorbent article 10 may also comprise one or more pairs of barrier leg cuffs 32 with or without elastics 33, one or more pairs of leg elastics 34, one or more waistbands 36, and/or one or more acquisition materials 38. The acquisition material or materials 38 may be positioned intermediate the topsheet 26 and the absorbent core 30. An outer cover 40, such as a nonwoven material, covers a garment-facing side of the backsheet 28. The absorbent article 10 may comprise back ears 42 in the back waist region 16. The back ears 42 may comprise fasteners 46 and may extend from the back waist region 16 of the absorbent article 10 and attach (using the fasteners 46) to the landing zone area or landing zone material 44 on a garment-facing portion of the front waist region 12 of the absorbent article 10. The absorbent article 10 may also have front ears 47 in the front waist region 12. The absorbent article 10 has a central lateral (or transverse) axis 48 and a central longitudinal axis 50. The central lateral axis 48 extends perpendicular to the central longitudinal axis 50.

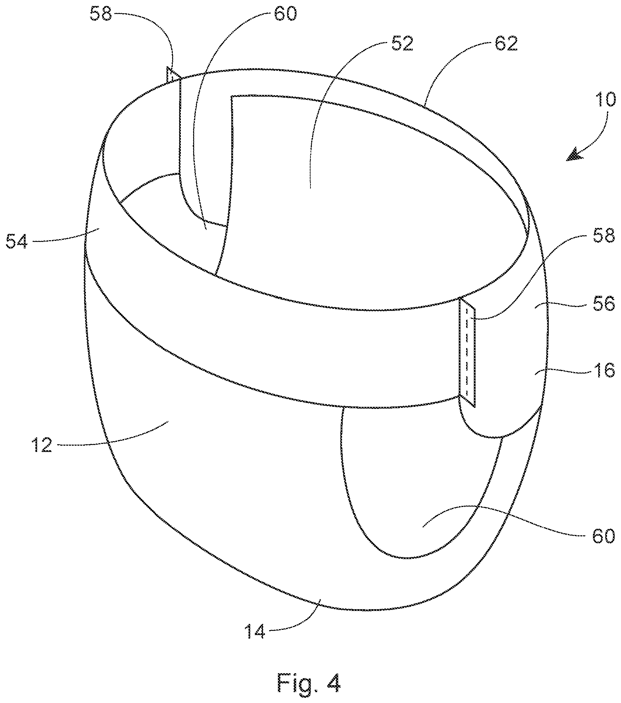

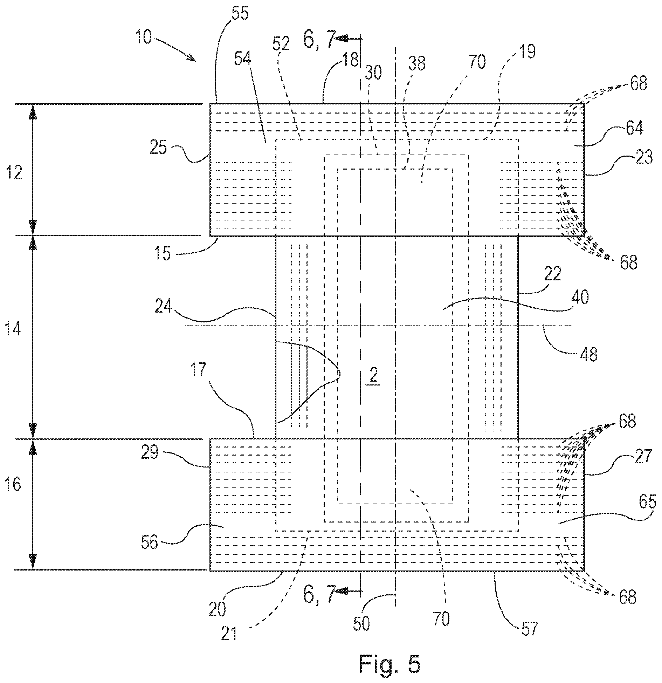

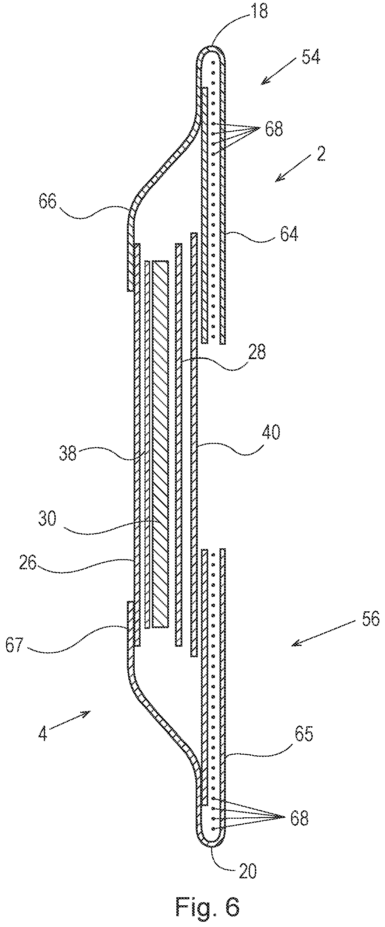

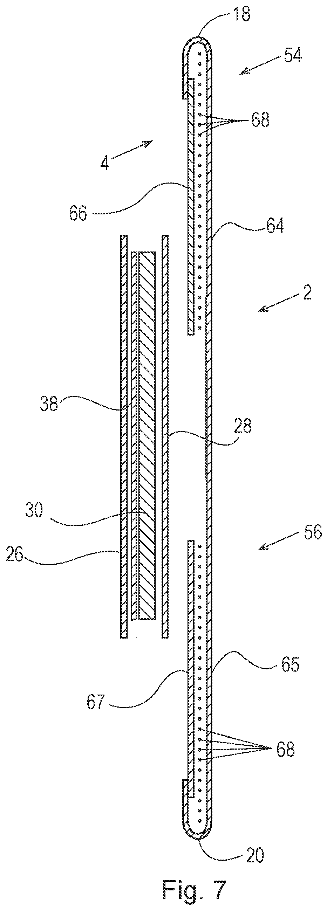

In other instances, the absorbent article may be in the form of a pant having permanent or refastenable side seams. Suitable refastenable seams are disclosed in U.S. Pat. Appl. Pub. No. 2014/0005020 and U.S. Pat. No. 9,421,137. Referring to Figs. 4-7, an example absorbent article 10 in the form of a pant is illustrated. Fig. 4 is a front perspective view of the absorbent article 10. Fig. 5 is a plan view of the absorbent article 10, laid flat, with the garment-facing surface facing the viewer. Elements of Fig. 4-7 having the same reference number as described above with respect to Figs. 1-3 may be the same element (e.g., absorbent core 30). Fig. 6 is an example cross-sectional view of the absorbent article taken about line 6-6 of Fig. 5. Fig. 7 is an example cross-sectional view of the absorbent article taken about line 7-7 of Fig. 5. Figs. 6 and 7 illustrate example forms of front and back belts 54, 56. The absorbent article 10 has a front waist region 12, a crotch region 14, and a back waist region 16. Each of the regions 12, 14, and 16 may be 1/3 of the length of the absorbent article 10. The absorbent article 10 has a chassis 52 (sometimes referred to as a central chassis or central panel) comprising a topsheet 26, a backsheet 28, and an absorbent core 30 disposed at least partially intermediate the topsheet 26 and the backsheet 28, and an optional acquisition material 38, similar to that as described above with respect to Figs. 1-3. The absorbent article 10 may comprise a front belt 54 in the front waist region 12 and a back belt 56 in the back waist region 16. The chassis 52 may be joined to a wearer-facing surface 4 of the front and back belts 54, 56 or to a garment-facing surface 2 of the belts 54, 56. Side edges 23 and 25 of the front belt 54 may be joined to side edges 27 and 29, respectively, of the back belt 56 to form two side seams 58. The side seams 58 may be any suitable seams known to those of skill in the art, such as butt seams or overlap seams, for example. When the side seams 58 are permanently formed or refastenably closed, the absorbent article 10 in the form of a pant has two leg openings 60 and a waist opening circumference 62. The side seams 58 may be permanently joined using adhesives or bonds, for example, or may be refastenably closed using hook and loop fasteners, for example.

### Belts

Referring to Figs. 5-7, the front and back belts 54 and 56 may comprise front and back inner belt layers 66 and 67 and front and back outer belt layers 64 and 65 having an elastomeric material (e.g., strands 68 or a film (which may be apertured)) disposed at least partially therebetween. The elastic elements 68 or the film may be relaxed (including being cut) to reduce elastic strain over the absorbent core 30 or, may alternatively, run continuously across the absorbent core 30. The elastics elements 68 may have uniform or variable spacing therebetween in any portion of the belts. The elastic elements 68 may also be pre-strained the same amount or different amounts. The front and/or back belts 54 and 56 may have one or more elastic element free zones 70 where the chassis 52 overlaps the belts 54, 56. In other instances, at least some of the elastic elements 68 may extend continuously across the chassis 52.

The front and back inner belt layers 66, 67 and the front and back outer belt layers 64, 65 may be joined using adhesives, heat bonds, pressure bonds or thermoplastic bonds. Various suitable belt layer configurations can be found in U.S. Pat. Appl. Pub. No. 2013/0211363.

Front and back belt end edges 55 and 57 may extend longitudinally beyond the front and back chassis end edges 19 and 21 (as shown in Fig. 5) or they may be coterminous. The front and back belt side edges 23, 25, 27, and 29 may extend laterally beyond the chassis side edges 22 and 24. The front and back belts 54 and 56 may be continuous (i.e., having at least one layer that is continuous) from belt side edge to belt side edge (e.g., the transverse distances from 23 to 25 and from 27 to 29). Alternatively, the front and back belts 54 and 56 may be discontinuous from belt side edge to belt side edge (e.g., the transverse distances from 23 to 25 and 27 to 29), such that they are discrete.

As disclosed in U.S. Pat. No. 7,901,393, the longitudinal length (along the central longitudinal axis 50) of the back belt 56 may be greater than the longitudinal length of the front belt 54, and this may be particularly useful for increased buttocks coverage when the back belt 56 has a greater longitudinal length versus the front belt 54 adjacent to or immediately adjacent to the side seams 58.

The front outer belt layer 64 and the back outer belt layer 65 may be separated from each other, such that the layers are discrete or, alternatively, these layers may be continuous, such that a layer runs continuously from the front belt end edge 55 to the back belt end edge 57. This may also be true for the front and back inner belt layers 66 and 67 - that is, they may also be longitudinally discrete or continuous. Further, the front and back outer belt layers 64 and 65 may be longitudinally continuous while the front and back inner belt layers 66 and 67 are longitudinally discrete, such that a gap is formed between them - a gap between the front and back inner and outer belt layers 64, 65, 66, and 67 is shown in Fig. 6 and a gap between the front and back inner belt layers 66 and 67 is shown in Fig. 7.

The front and back belts 54 and 56 may include slits, holes, and/or perforations providing increased breathability, softness, and a garment-like texture. Underwear-like appearance can be enhanced by substantially aligning the waist and leg edges at the side seams 58 (see Fig. 4).

The front and back belts 54 and 56 may comprise graphics (see e.g., 78 of Fig. 1). The graphics may extend substantially around the entire circumference of the absorbent article 10 and may be disposed across side seams 58 and/or across proximal front and back belt seams 15 and 17; or, alternatively, adjacent to the seams 58, 15, and 17 in the manner described in U.S. Pat. No. 9,498, 389 to create a more underwear-like article. The graphics may also be discontinuous.

Alternatively, instead of attaching belts 54 and 56 to the chassis 52 to form a pant, discrete side panels may be attached to side edges of the chassis 22 and 24. Suitable forms of pants comprising discrete side panels are disclosed in U.S. Pat. Nos. 6,645,190; 8,747,379; 8,372,052; 8,361,048; 6,761,711; 6,817,994; 8,007,485; 7,862,550; 6,969,377; 7,497,851; 6,849,067; 6,893,426; 6,953,452; 6,840,928; 8,579,876; 7,682,349; 7,156,833; and 7,201,744.

### Topsheet

The topsheet 26 is the part of the absorbent article 10 that is in contact with the wearer's skin. The topsheet 26 may be joined to portions of the backsheet 28, the absorbent core 30, the barrier leg cuffs 32, and/or any other layers as is known to those of ordinary skill in the art. The topsheet 26 may be compliant, soft-feeling, and non-irritating to the wearer's skin. Further, at least a portion of, or all of, the topsheet may be liquid permeable, permitting liquid bodily exudates to readily penetrate through its thickness. A suitable topsheet may be manufactured from a wide range of materials, such as porous foams, reticulated foams, apertured plastic films, woven materials, nonwoven materials, woven or nonwoven materials of natural fibers (e.g., wood or cotton fibers), synthetic fibers or filaments (e.g., polyester or polypropylene or bicomponent PE/PP fibers or mixtures thereof), or a combination of natural and synthetic fibers. The topsheet may have one or more layers. The topsheet may be apertured (Fig. 2, element 31), may have any suitable three-dimensional features, and/or may have a plurality of embossments (e.g., a bond pattern). The topsheet may be apertured by overbonding a material and then rupturing the overbonds through ring rolling, such as disclosed in U.S. Patent No. 5,628,097, to Benson et al., issued on May 13, 1997 and disclosed in U.S. Pat. Appl. Publication No. US 2016/0136014 to Arora et al. Any portion of the topsheet may be coated with a skin care composition, an antibacterial agent, a surfactant, and/or other beneficial agents. The topsheet may be hydrophilic or hydrophobic or may have hydrophilic and/or hydrophobic portions or layers. If the topsheet is hydrophobic, typically apertures will be present so that bodily exudates may pass through the top sheet.

### Backsheet

The backsheet 28 is generally that portion of the absorbent article 10 positioned proximate to the garment-facing surface of the absorbent core 30. The backsheet 28 may be joined to portions of the topsheet 26, the outer cover 40, the absorbent core 30, and/or any other layers of the absorbent article by any attachment methods known to those of skill in the art. The backsheet 28 prevents, or at least inhibits, the bodily exudates absorbed and contained in the absorbent core 10 from soiling articles such as bedsheets, undergarments, and/or clothing. The backsheet is typically liquid impermeable, or at least substantially liquid impermeable. The backsheet may, for example, be or comprise a thin plastic film, such as a thermoplastic film having a thickness of about 0.012 mm to about 0.051 mm. Other suitable backsheet materials may include breathable materials which permit vapors to escape from the absorbent article, while still preventing, or at least inhibiting, bodily exudates from passing through the backsheet.

### Outer Cover

The outer cover (sometimes referred to as a backsheet nonwoven) 40 may comprise one or more nonwoven materials joined to the backsheet 28 and that covers the backsheet 28. The outer cover 40 forms at least a portion of the garment-facing surface 2 of the absorbent article 10 and effectively "covers" the backsheet 28 so that film is not present on the garment-facing surface 2. The outer cover 40 may comprise a bond pattern, apertures, and/or three-dimensional features.

### Absorbent Core

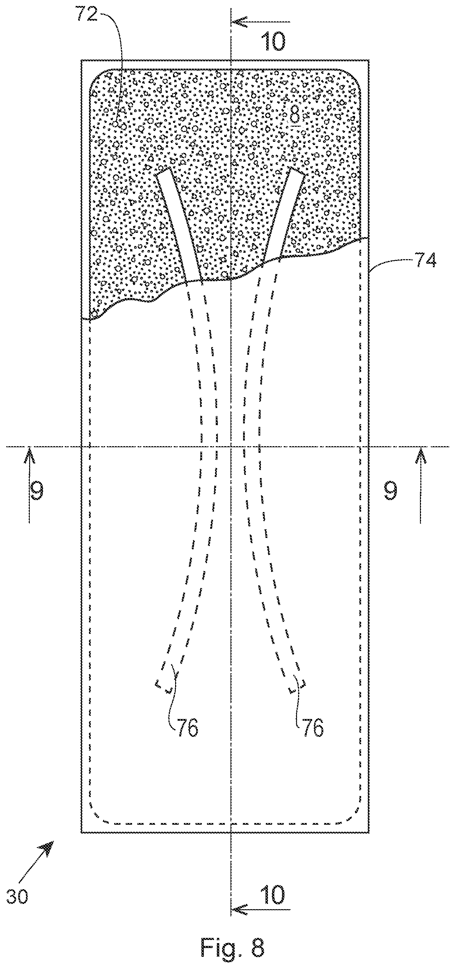

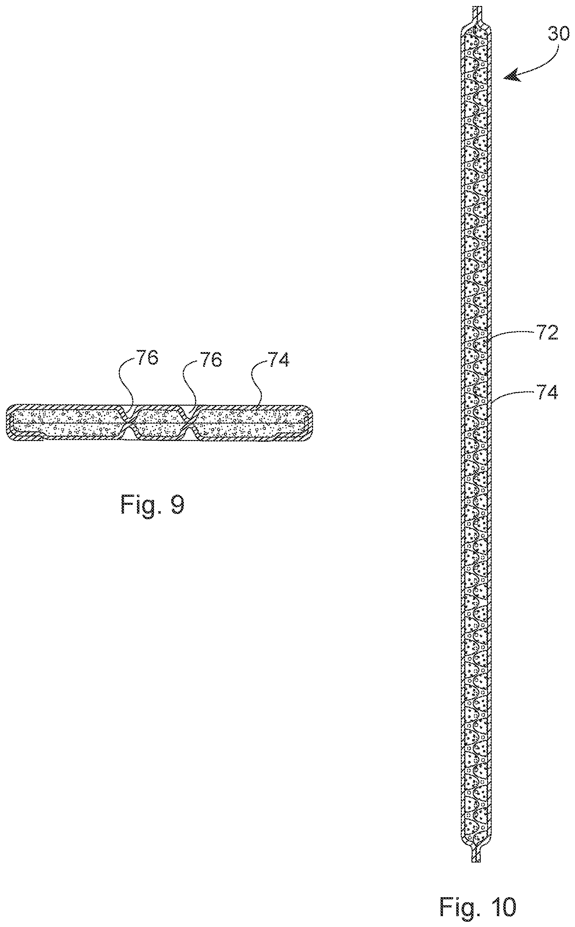

As used herein, the term "absorbent core" 30 refers to the component of the absorbent article 10 having the most absorbent capacity and that comprises an absorbent material. Referring to Figs. 8-10, in some instances, absorbent material 72 may be positioned within a core bag or a core wrap 74. The absorbent material may be profiled or not profiled, depending on the specific absorbent article. The absorbent core 30 may comprise, consist essentially of, or consist of, a core wrap, absorbent material 72, and glue enclosed within the core wrap. The absorbent material may comprise superabsorbent polymers, a mixture of superabsorbent polymers and air felt, only air felt, and/or a high internal phase emulsion foam. In some instances, the absorbent material may comprise at least 80%, at least 85%, at least 90%, at least 95%, at least 99%, or up to 100% superabsorbent polymers, by weight of the absorbent material. In such instances, the absorbent material may be free of air felt, or at least mostly free of air felt. The absorbent core periphery, which may be the periphery of the core wrap, may define any suitable shape, such as rectangular "T," "Y," "hour-glass," or "dog-bone" shaped, for example. An absorbent core periphery having a generally "dog bone" or "hour-glass" shape may taper along its width towards the crotch region 14 of the absorbent article 10.

Referring to Figs. 8-10, the absorbent core 30 may have areas having little or no absorbent material 72, where a wearer-facing surface of the core bag 74 may be joined to a garment-facing surface of the core bag 74. These areas having little or no absorbent material and may be referred to as "channels" 76. These channels can embody any suitable shapes and any suitable number of channels may be provided. In other instances, the absorbent core may be embossed to create the impression of channels. The absorbent core in Figs. 8-10 is merely an example absorbent core. Many other absorbent cores with or without channels are also within the scope of the present disclosure.

### Barrier Leg Cuffs/Leg Elastics

Referring to Figs. 1 and 2, for example, the absorbent article 10 may comprise one or more pairs of barrier leg cuffs 32 and one or more pairs of leg elastics 34. The barrier leg cuffs 32 may be positioned laterally inboard of leg elastics 34. Each barrier leg cuff 32 may be formed by a piece of material which is bonded to the absorbent article 10 so it can extend upwards from a wearer-facing surface 4 of the absorbent article 10 and provide improved containment of body exudates approximately at the junction of the torso and legs of the wearer. The barrier leg cuffs 32 are delimited by a proximal edge joined directly or indirectly to the topsheet and/or the backsheet and a free terminal edge, which is intended to contact and form a seal with the wearer's skin. The barrier leg cuffs 32 may extend at least partially between the front end edge 18 and the back end edge 20 of the absorbent article 10 on opposite sides of the central longitudinal axis 50 and may be at least present in the crotch region 14. The barrier leg cuffs 32 may each comprise one or more elastics 33 (e.g., elastic strands or strips) near or at the free terminal edge. These elastics 33 cause the barrier leg cuffs 32 to help form a seal around the legs and torso of a wearer. The leg elastics 34 extend at least partially between the front end edge 18 and the back end edge 20. The leg elastics 34 essentially cause portions of the absorbent article 10 proximate to the chassis side edges 22, 24 to help form a seal around the legs of the wearer. The leg elastics 34 may extend at least within the crotch region 14.

### Acquisition Materials

Referring to Figs. 1, 2, 6, and 7, one or more acquisition materials 38 may be present at least partially intermediate the topsheet 26 and the absorbent core 30. The acquisition materials 38 are typically hydrophilic materials that provide significant wicking of bodily exudates. These materials may dewater the topsheet 26 and quickly move bodily exudates into the absorbent core 30. The acquisition materials 38 may comprise one or more nonwoven materials, foams, cellulosic materials, cross-linked cellulosic materials, air laid cellulosic nonwoven materials, spunlace materials, or combinations thereof, for example. In some instances, portions of the acquisition materials 38 may extend through portions of the topsheet 26, portions of the topsheet 26 may extend through portions of the acquisition materials 38, and/or the topsheet 26 may be nested with the acquisition materials 38. Typically, an acquisition material 38 may have a width and length that are smaller than the width and length of the topsheet 26. The acquisition material may be a secondary topsheet in the feminine pad context. The acquisition material may have one or more channels as described above with reference to the absorbent core 30 (including the embossed version). The channels in the acquisition material may align or not align with channels in the absorbent core 30. In an example, a first acquisition material may comprise a nonwoven material and as second acquisition material may comprise a cross-linked cellulosic material.

### Landing Zone

Referring to Figs. 1 and 2, the absorbent article 10 may have a landing zone area 44 that is formed in a portion of the garment-facing surface 2 of the outer cover 40. The landing zone area 44 may be in the back waist region 16 if the absorbent article 10 fastens from front to back or may be in the front waist region 12 if the absorbent article 10 fastens back to front. In some instances, the landing zone 44 may be or may comprise one or more discrete nonwoven materials that are attached to a portion of the outer cover 40 in the front waist region 12 or the back waist region 16 depending upon whether the absorbent article fastens in the front or the back. In essence, the landing zone 44 is configured to receive the fasteners 46 and may comprise, for example, a plurality of loops configured to be engaged with, a plurality of hooks on the fasteners 46, or *vice versa.*

### Wetness Indicator/Graphics

Referring to Fig. 1, the absorbent articles 10 of the present disclosure may comprise graphics 78 and/or wetness indicators 80 that are visible from the garment-facing surface 2. The graphics 78 may be printed on the landing zone 40, the backsheet 28, and/or at other locations. The wetness indicators 80 are typically applied to the absorbent core facing side of the backsheet 28, so that they can be contacted by bodily exudates within the absorbent core 30. In some instances, the wetness indicators 80 may form portions of the graphics 78. For example, a wetness indicator may appear or disappear and create/remove a character within some graphics. In other instances, the wetness indicators 80 may coordinate (e.g., same design, same pattern, same color) or not coordinate with the graphics 78.

### Front and Back Ears

Referring to Figs. 1 and 2, as referenced above, the absorbent article 10 may have front and/or back ears 47, 42 in a taped diaper context. Only one set of ears may be required in most taped diapers. The single set of ears may comprise fasteners 46 configured to engage the landing zone or landing zone area 44. If two sets of ears are provided, in most instances, only one set of the ears may have fasteners 46, with the other set being free of fasteners. The ears, or portions thereof, may be elastic or may have elastic panels. In an example, an elastic film or elastic strands may be positioned intermediate a first nonwoven material and a second nonwoven material. The elastic film may or may not be apertured. The ears may be shaped. The ears may be integral (e.g., extension of the outer cover 40, the backsheet 28, and/or the topsheet 26) or may be discrete components attached to a chassis 52 of the absorbent article on a wearer-facing surface 4, on the garment-facing surface 2, or intermediate the two surfaces 4, 2.

### Sensors

Referring again to Fig. 1, the absorbent articles of the present disclosure may comprise a sensor system 82 for monitoring changes within the absorbent article 10. The sensor system 82 may be discrete from or integral with the absorbent article 10. The absorbent article 10 may comprise sensors that can sense various aspects of the absorbent article 10 associated with insults of bodily exudates such as urine and/or BM (e.g., the sensor system 82 may sense variations in temperature, humidity, presence of ammonia or urea, various vapor components of the exudates (urine and feces), changes in moisture vapor transmission through the absorbent articles garment-facing layer, changes in translucence of the garment-facing layer, and/or color changes through the garment-facing layer). Additionally, the sensor system 82 may sense components of urine, such as ammonia or urea and/or byproducts resulting from reactions of these components with the absorbent article 10. The sensor system 82 may sense byproducts that are produced when urine mixes with other components of the absorbent article 10 (e.g., adhesives, agm). The components or byproducts being sensed may be present as vapors that may pass through the garment-facing layer. It may also be desirable to place reactants in the absorbent article that change state (e.g. color, temperature) or create a measurable byproduct when mixed with urine or BM. The sensor system 82 may also sense changes in pH, pressure, odor, the presence of gas, blood, a chemical marker or a biological marker or combinations thereof. The sensor system 82 may have a component on or proximate to the absorbent article that transmits a signal to a receiver more distal from the absorbent article, such as an iPhone, for example. The receiver may output a result to communicate to the caregiver a condition of the absorbent article 10. In other instances, a receiver may not be provided, but instead the condition of the absorbent article 10 may be visually or audibly apparent from the sensor on the absorbent article.

### Packages

The absorbent articles of the present disclosure may be placed into packages. The packages may comprise polymeric films and/or other materials. Graphics and/or indicia relating to properties of the absorbent articles may be formed on, printed on, positioned on, and/or placed on outer portions of the packages. Each package may comprise a plurality of absorbent articles. The absorbent articles may be packed under compression so as to reduce the size of the packages, while still providing an adequate number of absorbent articles per package. By packaging the absorbent articles under compression, caregivers can easily handle and store the packages, while also providing distribution savings to manufacturers owing to the size of the packages.

### Arrays

"Array" means a display of packages comprising disposable absorbent articles of different article constructions (e.g., different elastomeric materials [compositionally and/or structurally] in the side panels, side flaps and/or belts flaps, different graphic elements, different product structures, fasteners or lack thereof). The packages may have the same brand and/or sub-brand and/or the same trademark registration and/or having been manufactured by or for a common manufacturer and the packages may be available at a common point of sale (e.g. oriented in proximity to each other in a given area of a retail store). An array is marketed as a line-up of products normally having like packaging elements (e.g., packaging material type, film, paper, dominant color, design theme, etc.) that convey to consumers that the different individual packages are part of a larger line-up. Arrays often have the same brand, for example, "Huggies," and same sub-brand, for example, "Pull-Ups." A different product in the array may have the same brand "Huggies" and the sub-brand "Little Movers." The differences between the "Pull-Ups" product of the array and the "Little Movers" product in the array may include product form, application style, different fastening designs or other structural elements intended to address the differences in physiological or psychological development. Furthermore, the packaging is distinctly different in that "Pull-Ups" is packaged in a predominately blue or pink film bag and "Little Movers" is packaged in a predominately red film bag.

Further regarding "Arrays," as another example an array may be formed by different products having different product forms manufactured by the same manufacturer, for example, "Kimberly-Clark", and bearing a common trademark registration for example, one product may have the brand name "Huggies," and sub-brand, for example, "Pull-Ups." A different product in the array may have a brand/sub-brand "Good Nites" and both are registered trademarks of The Kimberly-Clark Corporation and/or are manufactured by Kimberly-Clark. Arrays also often have the same trademarks, including trademarks of the brand, sub-brand, and/or features and/or benefits across the line-up. "On-line Array" means an "Array" distributed by a common on-line source.

### Waistband(s)

The disposable absorbent articles described herein intend to deliver a somewhat snug fit around a waist of a wearer. One way to achieve a snug fit around the waist of a wearer is to provide a waistband in at least one of the front waist region and/or the back waist region of the absorbent article chassis. The snug fit provided, at least in part, by a waistband or waistbands, may aid in sealing the absorbent article around a waist of a wearer to at least inhibit bodily exudates from escaping from the absorbent article through the waist opening circumference. In some instances, a waistband or waistbands may fully, or partially, surround the waist opening circumference of an absorbent article.

Referring to Figs. 1 and 2, the absorbent articles 10 of the present disclosure comprise one or more external waistband(s) 36. As discussed previously, an external waistband (a waistband disposed on a wearer-facing or garment-facing surface of a chassis of an absorbent article) may be less complicated and less costly to produce as compared to an internal waistband (a waistband disposed between layers of the chassis). The waistband(s) 36 may each comprise a waistband first end edge 37, a waistband second end edge 39, a waistband first side edge 41, and a waistband second side edge 43. The waistband first end edge 37 may be disposed more distal from the central lateral axis 48 than the waistband second end edge 39. The waistband first side edge 41 and second side edge 43 may be disposed on opposite sides of the central longitudinal axis 50. In some instances, the end edges and the side edges may be shaped, curved, or non-linear, for example. The waistband(s) 36 may be disposed in one of the front waist region 12 and/or the back waist region 16 of the chassis 52.

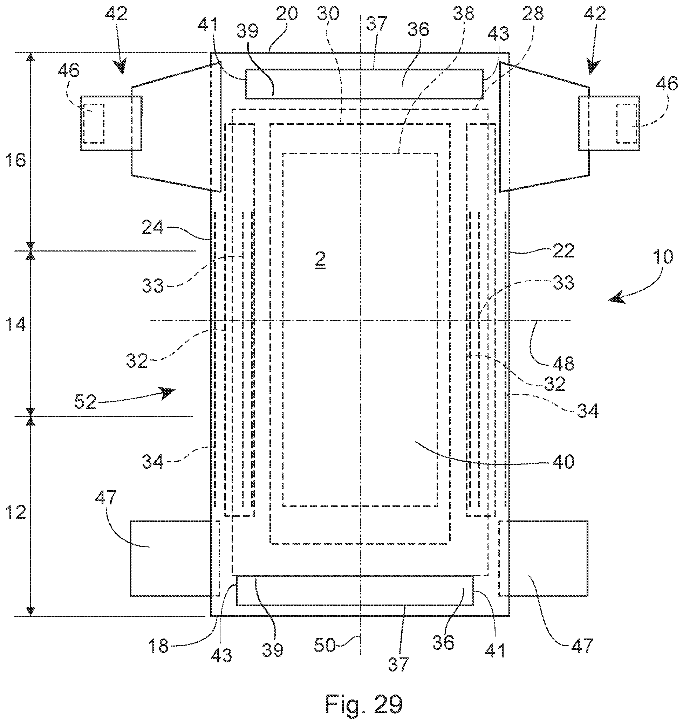

In one example, the waistband(s) 36 may be disposed on the wearer-facing surface of the topsheet 26 (as shown in Fig. 2). In another example, the waistband(s) 36 may be disposed on the garment-facing surface of the outer cover 40 (as shown in Fig. 29). In yet a further example, at least one waistband 36 may be disposed on the wearer-facing surface of the topsheet 26, and at least one other waistband 36 may be disposed on the garment-facing surface of the outer cover 40. In this example, both waistbands may be positioned in one waist region or both waistbands may be positioned in different waist regions. When an absorbent article comprises more than one waistband, the waistbands may all be the same in size and shape, may be similar in size and/or shape, or the waistbands may be different in size and/or shape. For example, a first waistband may have a longitudinal length measured in a direction parallel to the central longitudinal axis 50 that is shorter than a longitudinal length (measured the same way) of a second waistband. An absorbent article comprising waistbands of different sizes and/or shapes may provide an improved overall fit, as the differently sized and/or shaped waistbands may conform to different portions of a wearer's body.

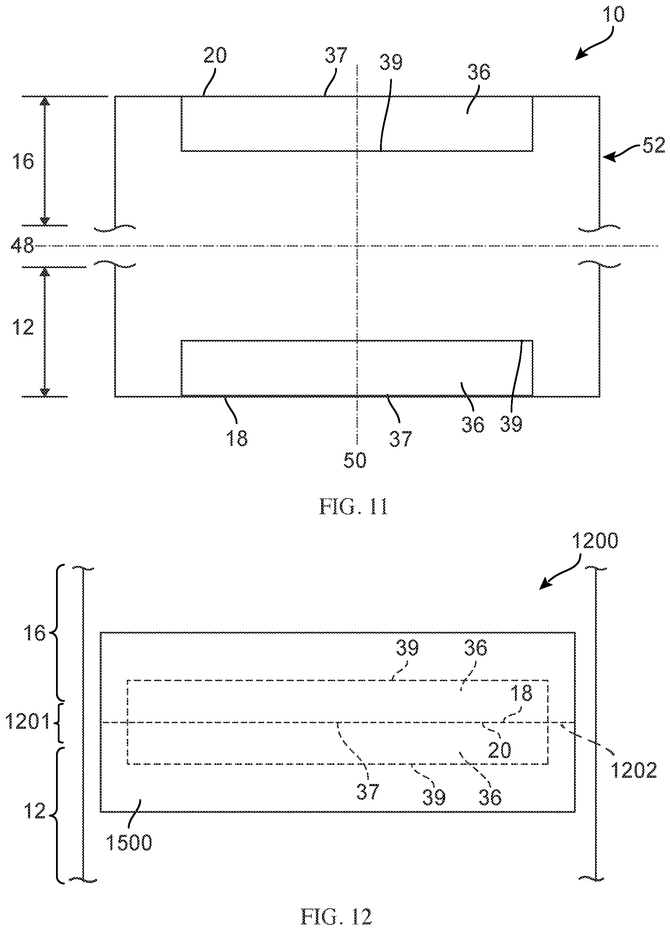

As shown in Figs. 1 and 2, the waistband(s) 36 may be offset from the front end edge 18 and/or the back end edge 20 of the chassis 52, meaning that a waistband first end edge 37 may be disposed more proximal to the central lateral axis 48 of the absorbent article 10 as compared to the front end edge 18 and/or back end edge 20 of the chassis 52. The waistband(s) 36 may be positioned inboard of the front end edge 18 and/or back end edge 20 by about 1mm to about 30mm, by about 1mm to about 20mm, by about 1mm to about 10mm, or about 1mm to about 5mm, for example. As shown in Fig. 11, a portion of, or all of, the one or more waistband(s) 36 may be coterminous with the front end edge 18 and/or the back end edge 20 of the chassis 52, meaning that at least a portion of, or all of, the first end edge 37 of waistband 36 is coincident with the front end edge 18 and/or the back end edge 20 of the chassis 52. An absorbent article comprising a waistband that is coterminous with a front end edge or a back end edge of the chassis may provide a benefit of easier and less costly manufacture, as cutting of the waistband may be subsequent to the waistband application to the article. For example, referring to Fig. 12, a waistband material may be placed in an article separation (cut) zone 1201 of a web of pre-cut absorbent articles 1200, such that a waistband 36 may be disposed on both the back waist region 16 of one absorbent article and on the front waist region 12 of another absorbent article. Only one knife cut 1202 may then be needed to separate the absorbent articles and the two waistbands 36. Therefore, fewer cuts may be required during production, placement of the first end edge 37 of the waistband 36 at the front end edge 18 and/or back end edge 20 may be more exact, and the overall production of the absorbent article may be simplified and lower cost.

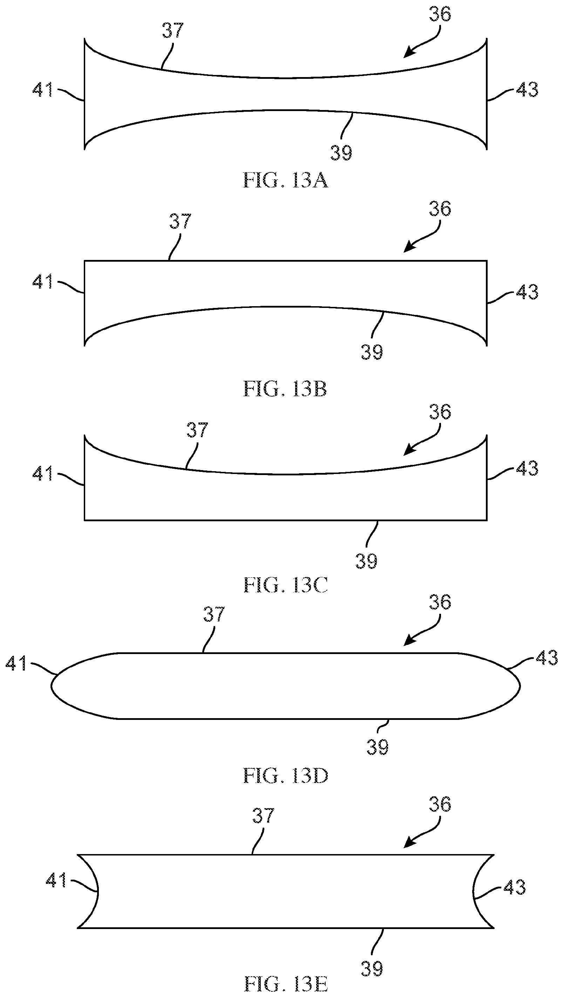

The waistband(s) 36 of the present disclosure may have a generally rectangular shape. Referring again to Figs. 1 and 2, the waistband first end edge 37 and second end edge 39 may or may not be generally parallel to each other, and may be longer than the waistband first side edge 41 and second side edge 43. The waistband(s) 36 may also be non-rectangular. Referring to Figs. 13A-E, the waistband(s) 36 of the present disclosure may have at least one side that is non-linear or shaped. As shown in Fig. 13A, the waistband 36 may comprise a waistband first end edge 37 and/or a waistband second edge 39 that is non-linear or shaped. For example, the waistband(s) 36 may comprise a waistband first end edge 37 and/or a waistband second edge 39 that is arcuate or that has arcuate portions. As shown in Figs. 13B and 13C, one waistband end edge 41, 43 may be generally linear, while the other waistband end edge may be non-linear, shaped, or arcuate. Referring to Figs. 13D and 13E, the waistband(s) 36 of the present disclosure may comprise at least one waistband first side edge 41 and/or waistband second side edge 43 that is non-linear, shaped, or arcuate. As shown in Fig. 13D, for example, the waistband first side edge 41 and/or second side edge 43 may be convex. In another example, as shown in Fig. 13E, the waistband first side edge 41 and/or second side edge 43 may be concave. The waistband may also take on any other suitable shapes.

The waistband(s) of the present disclosure may comprise an elastic material. In one example, the elastic material may comprise an elastic film or one or more elastic strands. In another example, the elastic material may comprise at least four elastic strands, at least six elastic strands, at least eight elastic strands, at least ten elastic strands, at least twelve elastic strands, but less than forty elastic strands. In a further example, the elastic material may comprise an elastomeric foam. The elastic material may be joined to the wearer-facing surface of the chassis or to the garment-facing surface of the chassis. In one example, the elastic material may be joined to the chassis by an adhesive. In another example, the elastic material may be joined to the chassis by a bond forming a portion of a bond pattern. Bonds may be formed by any known method in the art, including adhesives, thermal bonding, pressure bonding, ultrasonic bonding, and combinations thereof.

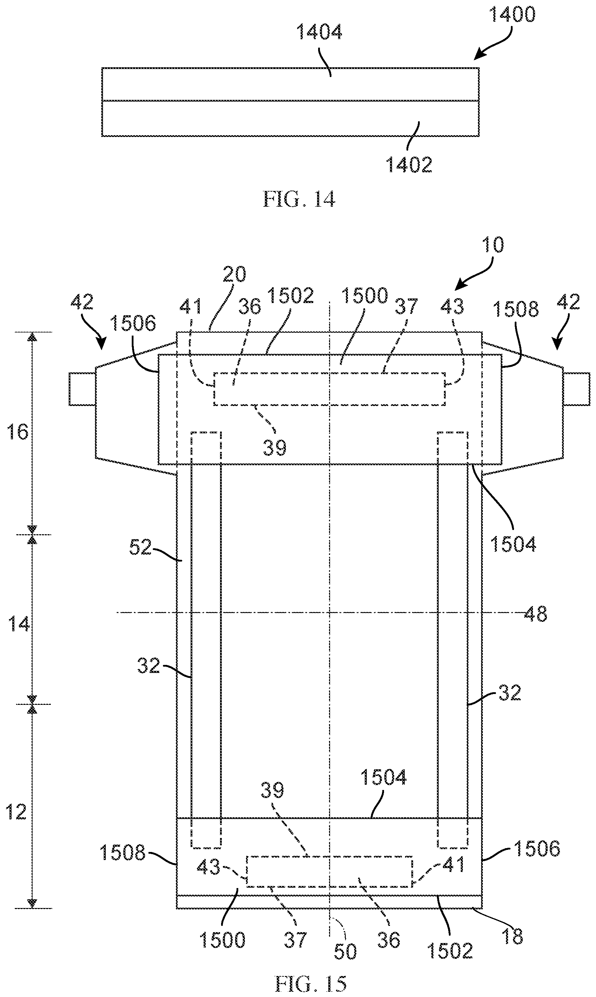

The waistband(s) of the present disclosure comprise a waistband laminate. Referring to Fig. 14, the waistband laminate 1400 comprises a substrate 1402 and an elastic material 1404. The substrate may comprise a wide range of materials, such as foams, plastic films, woven or nonwoven webs of natural fibers (e.g., wood or cotton fibers), synthetic fibers (e.g., polyester or polypropylene fibers), semi-synthetic fibers derived from cellulosic materials, such as rayon (including viscose, lyocell, and cuprammonium rayon), or combinations of natural, synthetic, and semi-synthetic fibers. The fibers of the woven and/or nonwoven webs may comprise mono-component, bi-component, and/or multi-component fibers. A nonwoven may comprise a spunbond nonwoven, an air-through nonwoven, a carded nonwoven, or other suitable nonwoven. Woven and nonwoven webs may have a basis weight of between about 9 gsm and about 55 gsm, between about 12 gsm and about 40 gsm, or between about 15 gsm and about 30 gsm. In one example, the waistband laminate substrate may comprise a 15-50 gsm, side-by-side polyethylene/polypropylene nonwoven web.

The waistband(s) of the present disclosure may be joined to a portion of the garment-facing surface or a portion of the wearer-facing surface of the absorbent article under an Applied Waistband Strain. The Applied Waistband Strain is the strain that the waistband is under when joined to the absorbent article. For example, if 100mm of waistband material is stretched to 170mm when applied to the absorbent article, the Applied Waistband Strain would be considered to be 70% or ((170mm - 100mm)/100mm x 100). The waistband(s) may be applied to the absorbent article at an Applied Waistband Strain of between about 20% and about 200%, between about 50% and about 150%, or between about 70% and about 125%. The waistband(s) may be joined to a portion of the chassis by, for example, a continuous bond, a series of discontinuous bonds, or a combination thereof. Bonds may be formed by any known method in the art, including by use of adhesives, thermal bonding, pressure bonding, ultrasonic bonding, and combinations thereof.

### Waistband Cover

Referring to Fig. 15, the absorbent articles of the present disclosure comprise one or more waistband covers 1500. A waistband cover 1500 may be provided for each waistband or a waistband cover may be provided for one waistband. As discussed previously, the waistband cover(s) 1500 may reduce the amplitude of local pressure concentrations on the skin of a wearer from, for example, exposed edges and ridges of waistband(s) 36. The waistband cover(s) 1500 may also reduce the incidence and/or severity of red marking on the skin of the wearer. In addition, the waistband cover(s) 1500 may provide a more finished, integrated, and/or softer appearance to an absorbent article 10 comprising external waistbands 36. For example, the waistband cover(s) 36 may overlap one, or more than one, end edge 37, 39 and/or side edge 41, 43 of a waistband and/or material edge of other elements. This overlap may provide a more finished, integrated, and/or softer appearance to the absorbent article because the continuous waistband cover(s) 1500 may overlap and hide from the sight of the user the material edges of the underlying elements. Therefore, there may be fewer material edges to break the continuity of the surface of the absorbent article and distract the eye of the user. Also, exposed material edges on a wearer-facing surface or garment-facing surface may be perceived as rough against the skin or garments of a wearer. Thus, while the absorbent article may be comprised of various underlying elements, the user may only notice the continuous surface of the waistband cover, and therefore perceive the article as more finished, integrated, and/or softer.

The waistband cover(s) 1500 comprises a waistband cover first end edge 1502 and a waistband cover second end edge 1504. The waistband cover first end edge 1502 is disposed more distal from the central lateral axis 48 than the waistband cover second end edge 1504. The waistband cover(s) 1500 also comprises a first side edge 1506 and a second side edge 1508, wherein the first and second side edges 1506, 1508 are disposed on opposite sides of the central longitudinal axis 50.

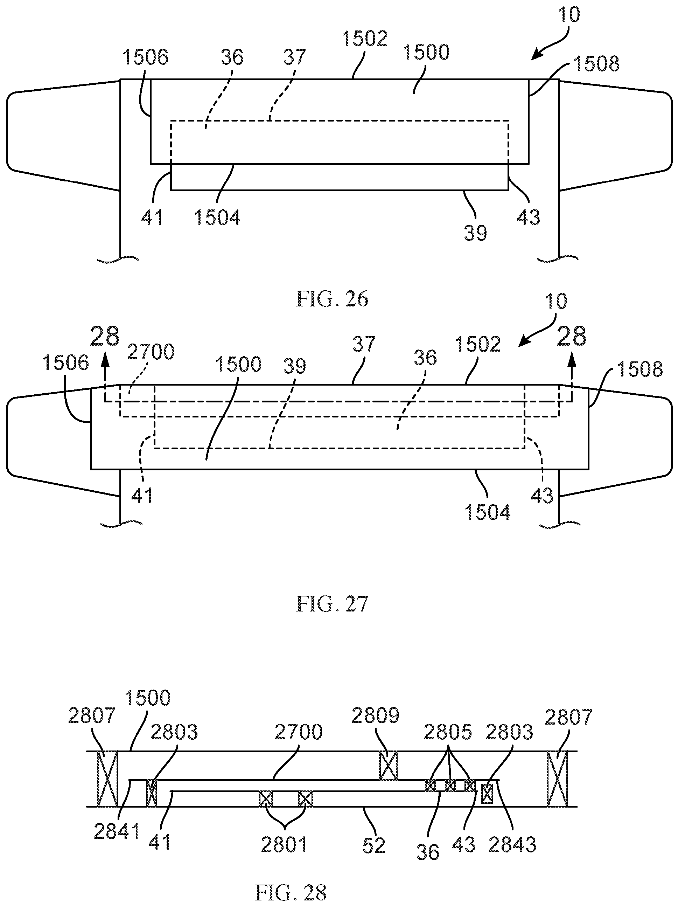

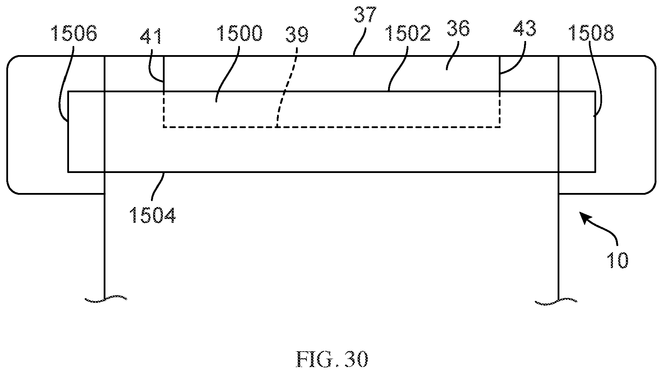

Referring again to Fig. 15, the absorbent articles 10 of the present disclosure may comprise one or more external waistband(s) 36. The waistband cover(s) 1500 may overlap a portion of, or all of, the one or more external waistband(s) 36. For example, the waistband cover(s) 1500 may overlap at least a portion of, or all of, the waistband(s) 36 first end edge 37, second end edge 39, first side edge 41, or second side edge 43. In another example, the waistband cover(s) 1500 may overlap at least a portion of, or all of, two of the waistband(s) 36 first end edge 37, second end edge 39, first side edge 41, and/or second side edge 43. In yet another example, as shown in Figs. 26 and 30, the waistband cover(s) 1500 may overlap at least a portion of, or all of, three of the waistband(s) 36 first end edge 37, second end edge 39, first side edge 41, and/or second side edge 43. As shown in Fig. 26, the waistband cover(s) 1500 may overlap at least a portion of, or all of, the first end edge 37, the first side edge 41, and the second side edge 43 of the waistband(s) 36. As shown in Fig. 30, the waistband cover(s) 1500 may overlap at least a portion of, or all of, the second end edge 37, the first side edge 41, and the second side edge 43 of the waistband(s) 36. In a further example, as shown in Fig. 15, the waistband cover(s) 1500 may overlap the entire waistband(s) 36 and/or all of the edges.

The waistband cover(s) 1500 overlaps a portion of, or all of, the one or more waistbands 36, such that only a portion of, or none of, the waistband(s) 36 form a portion of the wearer-facing surface or the garment-facing surface (taken together, the outer-facing surfaces) of the absorbent article 10. For example, a single waistband cover 1500 may overlap between about 10% and about 100%, between about 25% and about 100%, between about 45% and about 100%, or between about 60% and about 100% of an underlying waistband 36. When two waistband covers 1500 are provided, placement of a first waistband cover relative to a first waistband may be the same as, or different than, placement of a second waistband cover relative to a second waistband.

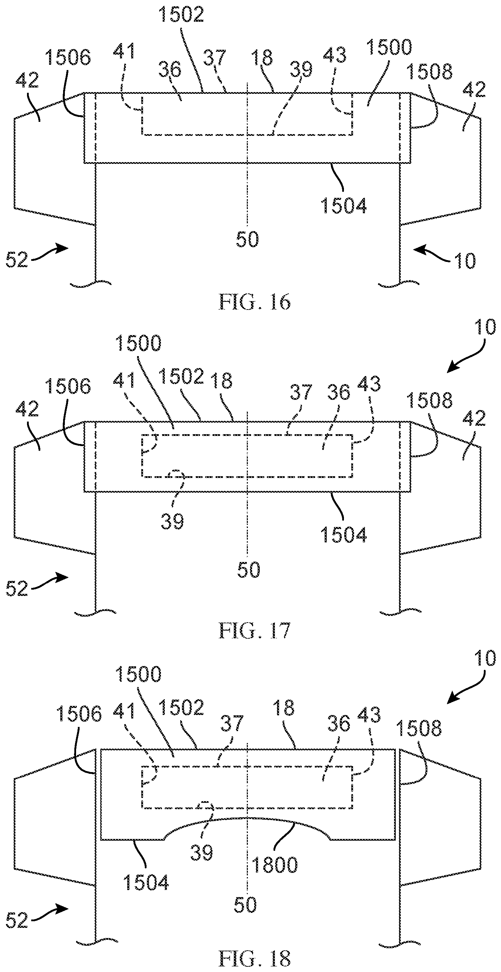

Placement of the waistband cover(s) 1500 on the chassis 52 may vary depending, in part, on the placement of the waistband(s) 36. For example, as shown in Fig. 15, the first end edge 37 of the waistband(s) 36 may be offset from the front and/or back end edges 18, 20 of the chassis 52. In such an example, the waistband cover(s) first end edge 1502 may likewise be offset from the front and/or back end edges 18, 20 of the chassis 52. In another example, as shown in Fig. 16, the first end edge 37 of the waistband(s) 36 may be coincident with the front and/or back end edges 18, 20 of the chassis 52 (i.e., the waistband(s) 36 may be coterminous with the front and/or back edges 18, 20 of the chassis 52), and the waistband cover first end edge 1502 may likewise be coincident with the front and/or back end edges 18, 20 of the chassis 52 (i.e., the waistband cover(s) 1500 may be coterminous with the front and/or back edges 18, 20 of the chassis 52). In a further example, as shown in Fig. 17, the first end edge 37 of the waistband(s) 36 may be offset from the front and/or back end edges 18, 20 of the chassis 52, while the waistband cover first end edge 1502 may be coincident with the front and/or back end edges 18, 20 of the chassis 52. An absorbent article comprising a waistband cover 1500, wherein the waistband cover 1500 is coterminous with the front and/or back edge 18, 20 of the chassis 52 may provide for a more integrated and finished look to the wearer-facing and/or garment-facing surface of the absorbent article because there may be fewer material edges to disrupt the continuity of inner or outer surface of the article. Also, as discussed previously, absorbent articles of the present disclosure may comprise waistbands of different sizes and/or shapes. As such, where an absorbent article comprises more than one waistband cover, the waistband covers may all be the same size and/or shape, may be a similar size and/or shape, or the waistband covers may be different sizes and/or shapes. The waistband cover(s) 1500 may have any suitable shape.

Referring again to Fig. 15, the waistband cover(s) 1500 may at least partially overlap portions of the one or more pairs of barrier leg cuffs 32. The waistband cover(s) first side edge 1506 and second side edge 1508 may be coincident with the side edges of the chassis 52, as illustrated by the waistband cover 1500 disposed in the front waist region 12 of the absorbent article 10 of Fig. 15. The waistband cover 1500 disposed in the back waist region 16 of the absorbent article 10 may at least partially overlap a portion of a pair of back ears 42. In an example, the waistband cover(s) 1500 may overlap at least a portion of the one or more waistbands 36 and a portion of the one or more pairs of barrier leg cuffs 32. In another example, the waistband cover(s) 1500 may overlap at least a portion of the one or more waistbands 36, a portion of the one or more pairs of barrier leg cuffs 32, and a portion of the pair of back ears 42. Overlap of the one or more pairs of barrier leg cuffs and/or the pair of back ears by the waistband cover(s) may provide for a more finished look to the wearer-facing surface of the absorbent article because there may be fewer material edges to break the continuity of the inner-surface of the absorbent article.

In some forms, the waistband cover(s) may have a generally rectangular shape. Referring again to Fig. 15, the waistband cover first end edge 1502 and second end edge 1504 may be generally parallel to each other, and may be longer than the waistband cover first side edge 1506 and second side edge 1508. The waistband cover(s) 1500 may also be non-rectangular and may have the same or similar shapes as discussed with respect to the waistbands of Figs. 13A-13E. Referring to Fig. 18, the waistband cover(s) second end edge 1504 may comprise a curvilinear portion 1800. The curvilinear portion 1800 may traverse the central longitudinal axis 50 of the absorbent article 10. The curvilinear portion 1800 may or may not be symmetrical about the central longitudinal axis 50. Waistband cover(s) comprising a curvilinear portion may provide a more gradual transition between the waistband cover(s) and the underlying portion of the absorbent article, thereby creating a more integrated appearance and feel.

The waistband cover(s) of the present disclosure may comprise a wide range of materials, such as foams, plastic films, woven or nonwoven webs of natural fibers (e.g., wood or cotton fibers), synthetic fibers (e.g., polyester or polypropylene fibers), semi-synthetic fibers derived from cellulosic materials, such as rayon (including viscose, lyocell, and cuprammonium rayon), elastomeric fibers, or combinations of natural, synthetic, semi-synthetic, and elastomeric fibers. Woven and nonwoven webs that comprise the waistband cover(s) may have a basis weight of between about 9 gsm and about 55 gsm, between about 12 gsm and about 40 gsm, or between about 15 gsm and about 30 gsm. The fibers of the woven and/or nonwoven webs may comprise mono-component, bi-component, and/or multi-component fibers. A nonwoven may comprise a spunbond nonwoven, an air-through nonwoven, a carded nonwoven, or other suitable nonwoven. In one example, the waistband cover(s) may comprise a 20 gsm, airthrough bonded, carded nonwoven web comprising 2.0 denier PE/PET bi-component fibers. A waistband cover comprising a nonwoven material with a low basis weight relative to the materials that comprise the rest of the absorbent article, for example between about 9 gsm and about 21 gsm, may have a relatively low opacity as compared to the other materials that make up the absorbent article. A waistband cover having a relatively low opacity may allow the underlying waistband to remain visible to the user, thereby communicating the benefit of an improved fit while still providing the benefits of the waistband cover. Where an absorbent article comprises more than one waistband cover, the waistband covers may comprise the same materials, or the waistband covers may comprise different materials.

The waistband cover(s) may comprise one or more layers, such as two layers of a spunbond material, a spunbond layer and a carded layer, or a spunbond-meltblown-spunbond material, for example. When the waistband cover(s) comprise more than one layer, each layer of the waistband cover(s) may be made of the same material, or one or more layers may be made of a different material. One layer may have a different hydrophobicity than the other layer, or they may be the same. One layer may have a different basis weight than the other layer, or they may be the same. In one example, a first wearer-facing layer may be hydrophobic and may define a plurality of apertures to allow bodily exudates to penetrate into a second, hydrophilic layer. As another example, the first layer may comprise carded cotton fibers and the second layer may comprise mono-component or bi-component continuous fibers. In yet another example, the wearer-facing layer may be hydrophilic and may assist in the removal of moisture (e.g., urine, sweat) from the surface of the skin of the wearer.

The waistband cover(s) of the present disclosure may comprise three-dimensional features such as bumps, ridges, protrusions, and/or recesses, such as those disclosed in U.S. Pat. No. 10,271,997 B2, issued to Arizti et al. on April 30, 2019, and morphological treatments such as those disclosed in U.S. Pat. Appl. Pub. No. 2017/0027774 A1, published in the name of Ashraf on February 2, 2017.

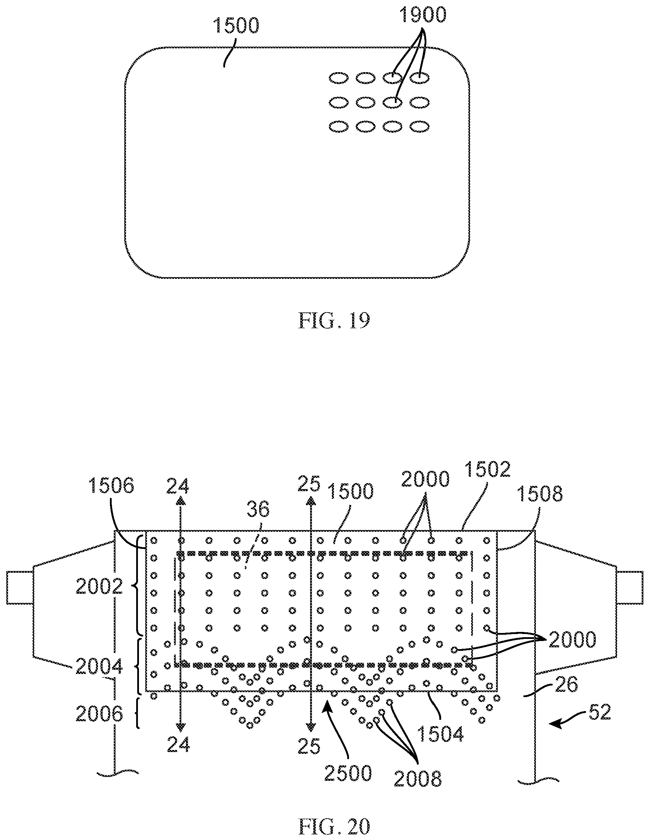

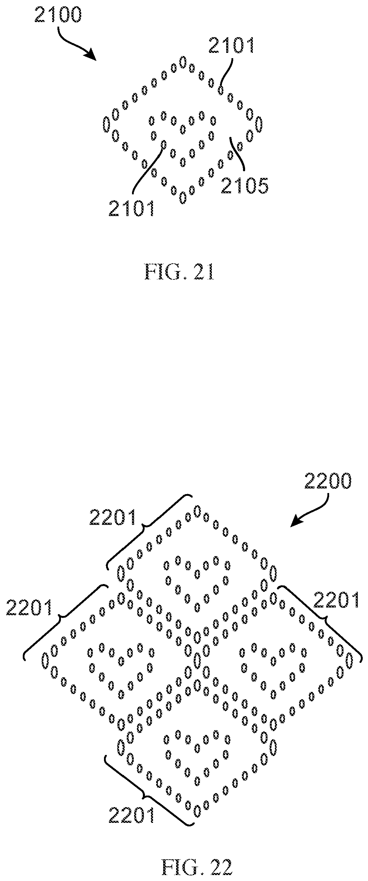

The waistband cover(s) of the present disclosure comprises a plurality of bonds forming a bond pattern or multiple bond patterns. The plurality of bonds may be homogenous in size and/or shape, or the bonds making up the plurality of bonds may vary in size and/or shape from one another. The plurality of bonds may comprise distinct individual bonds, for example, a first bond, a second, a third bond, etc. Bonds may be formed in the waistband cover(s) by any known method in the art, including thermal bonding (such as thermal point bonding), pressure bonding, ultrasonic bonding, and combinations thereof. In an example, where the waistband cover(s) comprises multiple layers of material, the waistband cover(s) may comprise a plurality of bonds that form a bond pattern and function to hold the multiple layers of material together. Referring to Fig. 19, the waistband cover(s) 1500 of the present disclosure comprise a plurality of bonds 1900. The plurality of bonds 1900 may be homogenous in size and/or shape or may be non-homogenous in size and/or shape. Referring now to Fig. 20, the waistband cover(s) comprises a first plurality of bonds 2000, wherein the first plurality of bonds 2000 forms a first bond pattern 2002. The first plurality of bonds 2000 may form a second, and different, bond pattern 2004 in addition to a first bond pattern 2002. The first plurality of bonds 2000 may form a homogenous and consistent pattern or patterns, or the first plurality of bonds 2000 may form random, non-homogenous patterns. As shown in Fig. 21, the plurality of bonds 2101 may be grouped in arrays of bonds 2100. Referring to Fig. 21, bonds 2101 of a bond array 2100 may be spaced such that a pattern emerges both in the bonds 2101 and also in the surrounding land area 2105. The bond arrays 2100 may form a regular or recognizable shape, such as a heart shape, diamond shape, polygon, ellipse, arrow, chevron, and/or other shapes known in the pattern art. Referring to Fig. 22, multiple bond arrays 2201 may be organized into "macro-arrays" 2200, having a higher order structure. For example, as shown in Fig. 22, multiple bond arrays 2201 are organized into a macro-array 2200, forming the higher order structure of a diamond shape.

The waistband cover(s) of the present disclosure may define a plurality of apertures. The apertures may have the same or similar patterns as the bonds discussed in Figs. 19-22.

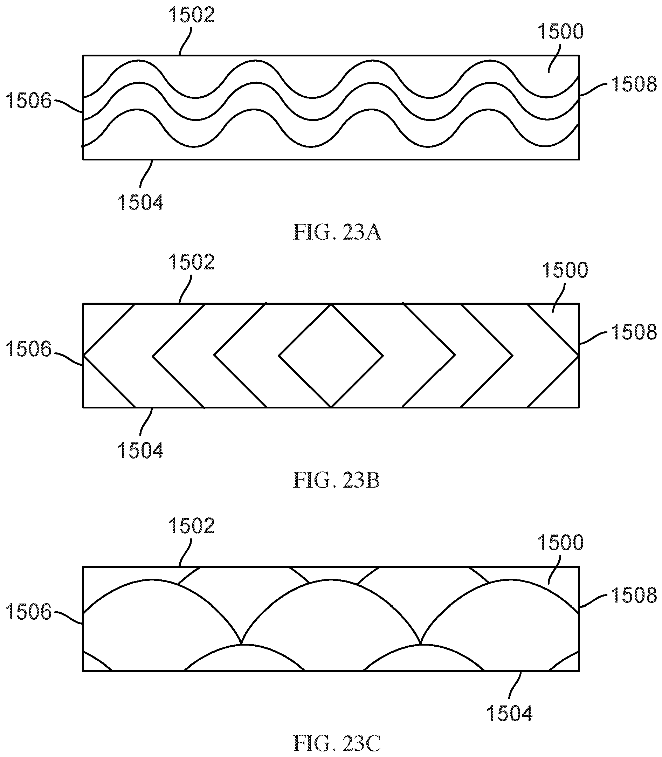

The waistband cover(s) of the present disclosure may comprise a pattern of color, patterns of embossment, and/or printed indicia. The pattern of color and/or printed indicia may comprise a single color or may comprise multiple colors (which includes shades of the same color e.g., dark blue and light blue). The pattern of color, embossment, and/or printed indicia may communicate to a user or caregiver indications of the functionality of the waistband cover(s) or of the absorbent article. For example, as shown in Fig. 23A, a printed sinusoidal wave pattern on the wearer-facing surface of the waistband cover(s) may signal to the user or caregiver that the absorbent article comprises an elastic waistband disposed under the waistband cover. In another example, a printed chevron pattern, as shown in Fig. 23B, may communicate to a user or caregiver that the portion of the absorbent article comprising the waistband cover 1500 may have lateral stretch properties. In a further example, as illustrated by Fig. 23C, a printed fan pattern disposed on the wearer-facing surface of the waistband cover(s) 1500 may signal to the user or caregiver that the waistband cover(s) 1500 is comprised of breathable materials. Where an absorbent article comprises more than one waistband cover, the waistband covers may comprise the same patterns of color, patterns of embossment, bond patterns, aperture patterns and/or printed indicia, or the waistband covers may comprise different patterns of color, patterns of embossment, bond patterns, aperture patterns and/or printed indicia.

Portions of the chassis may comprise coordinating and/or contrasting bond patterns, aperture patterns, color patterns, and/or printed indicia. Coordinating patterns are patterns that, when placed in close proximity to one another, appear to continue or complete a pattern. For example, referring to Fig. 20, the topsheet 26 may define a second plurality of bonds 2008 forming a third bond pattern 2006, wherein the third bond pattern 2006 coordinates with the second bond pattern 2004 of the waistband cover 1500, as the third bond pattern 2006 appears to complete the sinusoidal wave pattern of the second bond pattern 2004. The aperture patterns may be similar to or the same as described with respect to Fig. 20 and the bond patterns. Coordination of bond patterns, aperture patterns, colors, and/or printed indicia may allow the waistband cover(s) to appear more integrated into the chassis, and provide the appearance of a more finished absorbent article with fewer material edge lines noticeable to the user. On the other hand, contrasting bond patterns, aperture patterns, colors, and/or printed indicia may provide signals to a user or caregiver of the functionality of the waistband cover(s) or of the absorbent article.

The absorbent articles of the present disclosure comprise a waistband and a waistband cover. The waistband cover comprises a first plurality of bonds comprising a first bond. The first bond forms a portion of a first bond pattern. The first bond joins a portion of the waistband cover to a portion of the chassis and/or a portion of the waistband. The first bond may join a portion of the waistband cover to a portion of the topsheet or a portion of the outer cover. The waistband cover comprising the first plurality of bonds comprises a second bond. The second bond forms a portion of a second bond pattern. The second bond pattern is different than the first bond pattern. The second bond joins a portion of the waistband cover to a portion of the chassis and/or a portion of the waistband. The second bond may join a portion of the waistband cover to a portion of the topsheet or a portion of the outer cover. The first bond pattern may be disposed adjacent to a waistband cover first end edge. The second bond pattern may be disposed at least partially between a waistband cover second end edge and the first bond pattern. A portion of the chassis outside of the waistband cover comprises a third bond forming a portion of a third bond pattern. The third bond pattern is substantially similar tothe first and second bond patterns.

The waistband cover(s) of the present disclosure may be joined to underlying portions of the chassis, the back ears, and/or the waistband(s) by adhesives, thermal bonding, pressure bonding, ultrasonic bonding, combinations thereof, or any other bonding method known in the art. Attachment bonds, which are bonds that join the waistband cover(s) to underlying portions of the chassis, back ears, and/or waistband(s), may comprise a portion of, or all of, the bonds that form a bond pattern on the waistband cover(s), as discussed previously. Attachment bonds may be continuous, such as a continuous line of adhesive, or may be discontinuous, such as a series of discrete pressure bonds. The entire area of the waistband cover(s) may be continuously joined to underlying portions of the absorbent article, or only a portion of the waistband cover(s) may be joined to underlying portions of the absorbent article. For example, referring again to Fig. 20, all of the first plurality of bonds 2000 disposed on the waistband cover 1500 may be attachment bonds and may join the waistband cover 1500 to underlying portions of the absorbent article. **In** another example, only the bonds forming the first bond pattern 2002 may be attachment bonds and may join the waistband cover 1500 to underlying portions of the absorbent article, while the bonds forming the second bond pattern 2004 may be disposed only on the waistband cover 1500 and may not be attachment bonds. In a further example, only bonds disposed proximate to the waistband cover first end edge 1502, second end edge 1504, first side edge 1506, and second side edge 1508 may be attachment bonds and may join the waistband cover(s) to underlying portions of the absorbent article, while bonds offset from the edges of the waistband cover(s) may not be attachment bonds. In yet another example, only bonds disposed along the waistband cover first end edge 1502, first side edge 1506, and second side edge 1508 may be attachment bonds and may join the waistband cover(s) to underlying portions of the absorbent article 10, while bonds along at least a portion of the second end edge 1504, as well as bonds offset from the edges of the waistband cover(s), may not be attachment bonds. In such an example, the waistband cover(s) 1500 may form a waist pocket 2500, discussed further below.

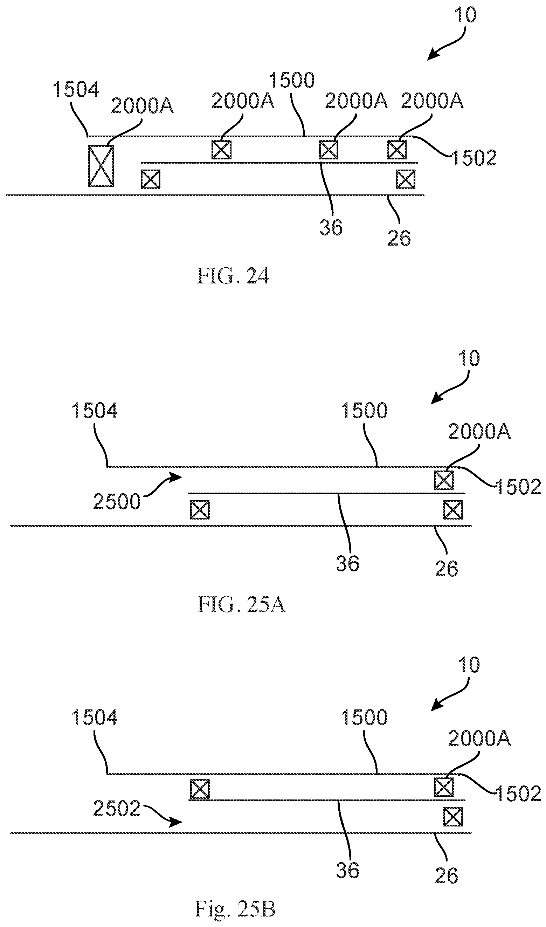

Figs. 24, 25A, and 25B are cross-sectional views of a portion of the absorbent article 10 taken at lines 24-24 (Fig. 24) and 25-25 (Figs. 25A and 25B) of Fig. 20, respectively. Referring to Fig. 24, attachment bonds 2000A may be disposed on the waistband cover 1500 only along the waistband cover first end edge 1502, waistband cover first side edge 1506, and waistband cover second side edge 1508. The attachment bonds 2000A may join the waistband cover 1500 to underlying portions of the absorbent article 10. Referring to Fig. 25A, at least a portion of the second end edge 1504 of the waistband cover(s) 1500 may be unattached to the underlying portions of absorbent article. In such an example, the waistband cover(s) 1500 may form a waist pocket(s) 2500. A waist pocket(s) 2500 may provide extra void volumes within the waist region(s) for containment of bodily exudates (e.g., fecal material). For example, when a wearer of an absorbent article has a bowel movement, many times the bowel movement spreads upon defecation and leaks out of the absorbent article in an area between the waist region and the wearer's back. Leaks of this type may happen, in part, because a snug fit between user's body and the donned absorbent article may not allow enough room for the runny bowel movement to remain contained within the absorbent article during the wearer's movement. As discussed previously, a snug fit of the absorbent article about the wearer may be enabled or enhanced by the incorporation of a waistband into the absorbent article. When the wearer moves, a portion of the bodily exudate may migrate into the waist pocket 2500, and may be held/trapped between the waistband cover 1500 and an underlying portion of the chassis 52 before it can leak out between the wearer's back and the back waist region of the absorbent article. Thus, the waist pocket 2500 may reduce leaks. Moreover, the waist pocket 2500 may help to trap fecal material and isolate it away from the wearer's skin. Referring to Fig. 25B, a waist pocket 2502 may be formed where a portion of the waistband 36 is unattached to the underlying absorbent article.

The waistband cover(s) may be joined to the underlying waistband(s) and/or portions of the chassis while the waistband(s) and chassis are under applied strain or in a relaxed state. Application of the waistband cover(s) to the absorbent article while the waistband(s) and chassis are under applied strain may result in the waistband cover(s) contracting with the waistband(s) upon relief of the applied strain, and the waistband(s) retaining at least a portion of their full stretch potential. On the other hand, application of the waistband cover(s) to the absorbent article while the waistband(s) are in a relaxed state may at least partially inhibit the stretch of the waistband(s). Inhibition of the waistband(s) stretch by the waistband cover(s) may produce a waistband with differential stretch characteristics. For example, referring to Fig. 26, the waistband cover 1500 partially overlaps the waistband 36. The waistband cover 1500 may be applied to the absorbent article while the waistband 36 is in a relaxed state. In such a case, the waistband cover 1500 may at least partially inhibit the stretch of the portion of the waistband 36 that it overlaps. The remainder of the waistband 36 may retain more, or all, of its ability to stretch to its maximum length. The differential stretch created by such a structure may allow the absorbent article to better fit around the waist and buttock of the wearer, where more stretch may be desired in one portion of the article as compared to another portion of the article.

Differential stretch characteristics of the waistband(s) may also be achieved by joining a portion of the waistband cover(s) to the underlying waistband(s), while leaving a portion of the waistband(s) free from attachment to the waistband cover(s). Attachment of the waistband cover(s) directly to the underlying waistband(s) may at least partially inhibit, or deaden, the elastic characteristic of the waistband(s). Referring again to Fig. 20, for example, the first plurality of bonds 2000 forming a first bond pattern 2002 may be attachment bonds, attaching the waistband cover 1500 to an underlying waistband. The first plurality of bonds 2000 forming the second bond pattern 2004 may not be attachment bonds, and may not attach the waistband cover 1500 to the underlying waistband. Without wishing to be bound by theory, it is believed that the portion of the waistband underlying the waistband cover 1500 comprising the first plurality of bonds 2000 forming the first bond pattern 2002 (the attachment bonds) may be inhibited from stretching to its maximum length because it may be restrained by the waistband cover 1500 that may exhibit less stretch and/or because the elastic characteristic of the waistband 36 may be at least partially deadened by the attachment bonds. The portion of the waistband underlying the waistband cover 1500 comprising the first plurality of bonds 2000 forming the second bond pattern 2004 and not attached to the waistband cover 1500, and may retain more, or all, of its elastic characteristic, thereby providing an absorbent article comprising a waistband comprising differential stretch characteristics.

### Stretch Inhibition Material

Differential stretch of the waistband(s) may also be achieved, in part, by placement of a stretch inhibition material overlapping a portion of a waistband. Referring to Fig. 27, the absorbent article 10 may comprise a waistband 36, a waistband cover 1500, and a stretch inhibition material 2700. The stretch inhibition material may be disposed between the waistband 36 and the waistband cover 1500, or between the waistband 36 and the chassis 52. The stretch inhibition material 2700 may overlap a portion of, or all of, the waistband 36. A portion of the waistband 36 may or may not be free from overlap with the stretch inhibition material 2700. The portion of the waistband 36 free from overlap with the stretch inhibition material 2700 may be a continuous portion of the waistband 36 from the waistband first side edge 41 to the waistband second side edge 43. The stretch inhibition material 2700 may be more resistant to stretch than the underlying portion of the waistband 36, and may therefore prevent, or at least inhibit, the underlying portion of the waistband 36 from stretching to its maximum length. In an example, the stretch inhibition material 2700 may be formed of a portion of a waistband cover 1500, may be attached to a waistband cover 1500, or may be a waistband cover 1500.

Fig. 28 is a cross-sectional view of the portion of the absorbent article 10 illustrated in Fig. 27 taken at line 28-28. As shown in Fig. 28, at least a portion of the waistband 36 may be joined to a portion of the chassis 52 by an attachment bond or series of attachment bonds 2801. The stretch inhibition material 2700 may overlap a portion of the waistband 36 and may extend laterally beyond the waistband first side edge 41 and the waistband second side edge 43. The stretch inhibition material 2700 may be joined directly to a portion of the chassis 52 laterally beyond the waistband first side edge 41 and second side edge 43 by an attachment bond or series of attachment bonds 2803. The stretch inhibition material 2700 may also be joined directly to the underlying portion of the waistband 36 by an attachment bond or series of attachment bonds 2805. For example, where the waistband comprises only an elastic material, the stretch inhibition material may be joined directly to the waistband elastic material. In another example, where the waistband comprises a waistband laminate, the stretch inhibition material may be joined to the waistband substrate, for example a nonwoven substrate. Without wishing to be bound by theory, it is believed that joining the stretch inhibition material 2700, which may be more resistant to stretch, directly to the waistband 36, the elastic characteristic of the waistband 36 may be at least partially inhibited. Still referring to Fig. 28, the stretch inhibition material 2700 may be disposed between the waistband 36 and the waistband cover 1500, or may be disposed between the chassis 52 and the waistband 36. The waistband cover 1500 may be joined directly to a portion of the chassis 52 laterally beyond a stretch inhibition material first side edge 2841 and a stretch inhibition material second side edge 2843 by an attachment bond or series of attachment bonds 2807. The waistband cover 1500 may also be joined directly to the stretch inhibition material 2700 by an attachment bond or series of attachment bonds 2809.

The stretch inhibition material of the present disclosure may comprise films, cellulosic materials, or nonwoven webs of natural fibers (e.g., wood or cotton fibers), synthetic fibers (e.g., polyester or polypropylene fibers), semi-synthetic fibers derived from cellulosic materials, such as rayon (including viscose, lyocell, and cuprammonium rayon), elastomeric fibers, or combinations of natural, synthetic, semi-synthetic, and elastomeric fibers. Nonwoven webs that comprise the stretch inhibition material may have a basis weight of between about 9 gsm and about 55 gsm, between about 12 gsm and about 40 gsm, or between about 15 gsm and about 30 gsm.

### Bio-Based Content for Components

Components of the absorbent articles, including the waistband cover(s) and stretch inhibition material(s) described herein, may at least partially be comprised of bio-based content as described in U.S. Pat. Appl. No. 2007/0219521 A1. For example, the superabsorbent polymer component may be bio-based via their derivation from bio-based acrylic acid. Bio-based acrylic acid and methods of production are further described in U.S. Pat. Appl. Pub. No. 2007/0219521 and U.S. Pat. Nos. 8,703,450; 9,630,901 and 9,822,197. Other components, for example nonwoven and film components, may comprise bio-based polyolefin materials. Bio-based polyolefins are further discussed in U.S. Pat. Appl. Pub. Nos. 2011/0139657, 2011/0139658, 2011/0152812, and 2016/0206774, and U.S. Pat. No. 9,169,366. Example bio-based polyolefins for use in the present disclosure comprise polymers available under the designations SHA7260^{™}, SHE150^{™}, or SGM9450F^{™} (all available from Braskem S.A.).

An absorbent article component may comprise a bio-based content value from about 10% to about 100%, from about 25% to about 100%, from about 40% to about 100%, from about 50% to about 100%, from about 75% to about 100%, or from about 90% to about 100%, for example, using ASTM D6866-10, method B.

### Test Method

Unless indicated otherwise, all tests described herein are made with samples conditioned at least 24 hours at 23 °C ± 2 °C and 50% ± 10% Relative Humidity (RH).

### Basis Weight Test:

The basis weight of a nonwoven material of interest may be determined by several available techniques. A simple representative technique involves first excising that nonwoven from an absorbent article or other consumer product. The nonwoven is separated from any and all adjacent components (e.g. elastics) and layers. If nonwoven is adhesively bonded to elastics or to other nonwovens, films, or substrates, removal of the nonwoven material from other layers may be aided by the use of cryogenic spray (such as Cyto-Freeze, Control Company, Houston, Texas, or equivalent). If excess adhesive is present on the nonwoven is after excision, an appropriate solvent (e.g. THF, hexanes, etc.) may be used to remove residual adhesive present. Five like specimens of nonwoven are excised from the corresponding location in each of five like absorbent articles or other consumer products.

Each of the five nonwoven specimens is cut to a produce a specimen portion with precisely known area for subsequent mass determination. (Only pristine nonwoven, undamaged during excision, can be used henceforth.) This is easily facilitated via the use of a steel rule die punch of known area (such as a circle with diameter 10 mm and area 78.5 mm²). In order to increase measurement precision, multiple punched areas from a single specimen nonwoven may be produced and then weighed together as a specimen portion. If, for example, three punches of a circle 10 mm in diameter were taken from the overall specimen, the area of the resulting specimen portion used subsequently would be 78.5 mm² × 3 = 236 mm².

An analytical four-place balance is used to determine the mass of each of the five specimen portions of precisely known area, and the mass of each is recorded to the nearest 0.0001 g. The basis weight of each specimen is then determined using the known area of each specimen portion, and the basis weight of each is recorded to the nearest 0.1 gram per square meter (gsm). Finally, the arithmetic mean of the five specimen basis weights is calculated and reported to the nearest integer in units of gsm as the basis weight of the nonwoven of interest.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. An absorbent article (10) comprising:
a chassis (52) comprising:
a central longitudinal axis (50),
a central lateral axis (48),
a liquid permeable topsheet (26),
a liquid impermeable backsheet (28),
an absorbent core (30) disposed at least partially between the topsheet (26) and the backsheet (28),
an outer cover (40),
a front waist region (12) comprising a front end edge (18),
a back waist region (16) comprising a back end edge (20), and
a crotch region (14) disposed between the front waist region (12) and the back waist region (16);
a waistband laminate (1400) comprising a first substrate (1402), an elastic material (1404), a waistband laminate first end edge, a waistband laminate second end edge, a waistband laminate first side edge, and a waistband laminate second side edge, wherein the waistband laminate first end edge is disposed more distal from the central lateral axis (48) than the waistband laminate second end edge, wherein the waistband laminate first and second side edges are disposed on opposite sides of the central longitudinal axis (50), and wherein the waistband laminate is disposed in one of the front waist region (12) or the back waist region (16) of the chassis (52); and
a waistband cover (1500) comprising a waistband cover first end edge (1502) and a waistband cover second end edge (1504), wherein the waistband cover first end edge (1502) is disposed more distal from the central lateral axis (48) than the waistband cover second end edge (1504), wherein a portion of the waistband cover (1500) is joined to the chassis (52), and wherein the waistband cover (1500) overlaps at least a portion of one of the waistband laminate first end edge, the waistband laminate second end edge, the waistband laminate first side edge, and the waistband laminate second side edge;
wherein the waistband cover (1500) is joined to a portion of the chassis (52) and/or a portion of the waistband laminate (1400) by a first bond forming a portion of a first bond pattern (2002);
wherein the waistband cover (1500) is joined to a portion of the chassis (52) and/or a portion of the waistband laminate (1400) by a second bond forming a portion of a second bond pattern (2004), and wherein the second bond pattern (2004) is different than the first bond pattern (2002); and
wherein a portion of the chassis(52) outside of the waistband cover (1500) comprises a third bond forming portion of a third bond pattern (2006), and wherein the third bond pattern (2006) is substantially similar to one of the first bond pattern (2002) and the second pattern (2004).

2. The absorbent article of claim 1, wherein the waistband cover (1500) completely overlaps an entire surface of waistband laminate (1400).

3. The absorbent article of claim 1, wherein the waistband cover (1500) is joined to a portion of the topsheet (26) or a portion of the outer cover (40) by the first bond forming a portion of the first bond pattern (2002).

4. The absorbent article of any one of the preceding claims, wherein a portion of the waistband cover second end edge is unattached to the chassis (52) at least in an area overlapping the central longitudinal axis (50) of the chassis (52).

5. The absorbent article of any one of the preceding claims, wherein the waistband cover (1500) defines a first plurality of apertures.

6. The absorbent article of claim 5, wherein the topsheet (26) and/or the outer cover (40) define(s) a second plurality of apertures, and wherein a first pattern formed by the first plurality of apertures coordinates with a second pattern formed by the second plurality of apertures.

7. The absorbent article of any one of the preceding claims, wherein the waistband cover first end edge is coincident with the front waist edge (18) or the back waist edge (20).

8. The absorbent article of any one of the preceding claims, comprising a pair of ears joined to and extending outwardly from the front waist region (12) and/or the back waist region (16), wherein a portion of the waistband cover (1500) is joined to each ear of the pair of ears.

## Patentansprüche

1. Absorptionsartikel (10), umfassend:
eine Grundeinheit (52), umfassend:
eine Mittellängsachse (50),
eine Mittelquerachse (48),
eine flüssigkeitsdurchlässige Oberschicht (26),
eine flüssigkeitsundurchlässige Unterschicht (28),
einen Absorptionskern (30), der wenigstens teilweise zwischen der Oberschicht (26) und der Unterschicht (28) angeordnet ist,
eine Außenabdeckung (40),
einen vorderseitigen Taillenbereich (12), umfassend einen vorderseitigen Endrand (18),
einen rückseitigen Taillenbereich (16), umfassend einen rückseitigen Endrand (20), und
einen Schrittbereich (14), der zwischen dem vorderseitigen Taillenbereich (12) und dem rückseitigen Taillenbereich (16) angeordnet ist;
ein Taillenbandlaminat (1400), umfassend ein erstes Substrat (1402), ein elastisches Material (1404), einen ersten Endrand des Taillenbandlaminats, einen zweiten Endrand des Taillenbandlaminats, einen ersten Seitenrand des Taillenbandlaminats und einen zweiten Seitenrand des Taillenbandlaminats, wobei der erste Endrand des Taillenbandlaminats distaler von der Mittelquerachse (48) als der zweite Endrand des Taillenbandlaminats angeordnet ist, wobei der erste und der zweite Seitenrand des Taillenbandlaminats auf einander entgegengesetzten Seiten der Mittellängsachse (50) angeordnet sind und wobei das Taillenbandlaminat in einem von dem vorderseitigen Taillenbereich (12) oder dem rückseitigen Taillenbereich (16) der Grundeinheit (52) angeordnet ist; und
eine Taillenbandabdeckung (1500), umfassend einen ersten Endrand (1502) der Taillenbandabdeckung und einen zweiten Endrand (1504) der Taillenbandabdeckung, wobei der erste Endrand (1502) der Taillenbandabdeckung distaler von der Mittelquerachse (48) als der zweite Endrand (1504) der Taillenbandabdeckung angeordnet ist, wobei ein Abschnitt der Taillenbandabdeckung (1500) an die Grundeinheit (52) gebunden ist und wobei die Taillenbandabdeckung (1500) wenigstens einen Abschnitt von einem von dem ersten Endrand des Taillenbandlaminats, dem zweiten Endrand des Taillenbandlaminats, dem ersten Seitenrand des Taillenbandlaminats und dem zweiten Seitenrand des Taillenbandlaminats überlappt;
wobei die Taillenbandabdeckung (1500) an einen Abschnitt der Grundeinheit (52) und/oder einen Abschnitt des Taillenbandlaminats (1400) durch eine erste Verbindung gebunden ist, die einen Abschnitt eines ersten Verbindungsmusters (2002) ausbildet;
wobei die Taillenbandabdeckung (1500) an einen Abschnitt der Grundeinheit (52) und/oder einen Abschnitt des Taillenbandlaminats (1400) durch eine zweite Verbindung gebunden ist, die einen Abschnitt eines zweiten Verbindungsmusters (2004) ausbildet, und wobei sich das zweite Verbindungsmuster (2004) von dem ersten Verbindungsmuster (2002) unterscheidet; und
wobei ein Abschnitt der Grundeinheit (52) außerhalb der Taillenbandabdeckung (1500) einen dritten verbindungsausbildenden Abschnitt eines dritten Verbindungsmusters (2006) umfasst und wobei das dritte Verbindungsmuster (2006) einem von dem ersten Verbindungsmuster (2002) und dem zweiten Muster (2004) im Wesentlichen ähnlich ist.

2. Absorptionsartikel nach Anspruch 1, wobei die Taillenbandabdeckung (1500) eine gesamte Oberfläche des Taillenbandlaminats (1400) vollständig überlappt.

3. Absorptionsartikel nach Anspruch 1, wobei die Taillenbandabdeckung (1500) an einen Abschnitt der Oberschicht (26) oder einen Abschnitt der Außenabdeckung (40) durch die erste Verbindung, die einen Abschnitt des ersten Verbindungsmusters (2002) ausbildet, gebunden ist.

4. Absorptionsartikel nach einem der vorstehenden Ansprüche, wobei ein Abschnitt des zweiten Endrands der Taillenbandabdeckung wenigstens in einem die Mittellängsachse (50) der Grundeinheit (52) überlappenden Gebiet nicht an der Grundeinheit (52) befestigt ist.

5. Absorptionsartikel nach einem der vorstehenden Ansprüche, wobei die Taillenbandabdeckung (1500) eine erste Vielzahl von Öffnungen bestimmt.

6. Absorptionsartikel nach Anspruch 5, wobei die Oberschicht (26) und/oder die Außenabdeckung (40) eine zweite Vielzahl von Öffnungen bestimmt und wobei ein durch die erste Vielzahl von Öffnungen ausgebildetes erstes Muster mit einem durch die zweite Vielzahl von Öffnungen ausgebildeten zweiten Muster abgestimmt ist.

7. Absorptionsartikel nach einem der vorstehenden Ansprüche, wobei der erste Endrand der Taillenbandabdeckung mit dem vorderseitigen Taillenrand (18) oder dem rückseitigen Taillenrand (20) zusammenfällt.

8. Absorptionsartikel nach einem der vorstehenden Ansprüche, umfassend ein Paar Seitenlappen, die an den vorderseitigen Taillenbereich (12) und/oder den rückseitigen Taillenbereich (16) gebunden sind und sich von diesen nach außen erstrecken, wobei ein Abschnitt der Taillenbandabdeckung (1500) an jeden Seitenlappen des Paares von Seitenlappen gebunden ist.

## Revendications

1. Article absorbant (10) comprenant :
un châssis (52) comprenant :
un axe longitudinal central (50),
un axe latéral central (48),
une feuille de dessus (26) perméable aux liquides,
une feuille de fond (28) imperméable aux liquides,
une âme absorbante (30) disposée au moins partiellement entre la feuille de dessus (26) et la feuille de fond (28),
une couverture externe (40),
une région de ceinture avant (12) comprenant un bord d'extrémité avant (18),
une région de ceinture arrière (16) comprenant un bord d'extrémité arrière (20), et
une région d'entrejambe (14) disposée entre la région de ceinture avant (12) et la région de ceinture arrière (16) ;
un stratifié de bande de ceinture (1400) comprenant un premier substrat (1402), un matériau élastique (1404), un premier bord extrême de stratifié de bande de ceinture, un second bord extrême de stratifié de bande de ceinture, un premier bord latéral de stratifié de bande de ceinture et un second bord latéral de stratifié de bande de ceinture, dans lequel le premier bord extrême de stratifié de bande de ceinture est disposé plus distal par rapport à l'axe latéral central (48) que le second bord extrême de stratifié de bande de ceinture, dans lequel les premier et second bords latéraux de stratifié de bande de ceinture sont disposés sur des côtés opposés de l'axe longitudinal central (50), et dans lequel le stratifié de bande de ceinture est disposé dans l'une de la région de bande de ceinture avant (12) ou de la région de bande de ceinture arrière (16) du châssis (52) ; et
une couverture de bande de ceinture (1500) comprenant un premier bord extrême de couverture de bande de ceinture (1502) et un second bord extrême de couverture de bande de ceinture (1504), dans lequel le premier bord extrême de couverture de bande de ceinture (1502) est disposé plus distal par rapport à l'axe latéral central (48) que le second bord extrême de couverture de bande de ceinture (1504), dans lequel une partie de la couverture de bande de ceinture (1500) est jointe au châssis (52), et dans lequel la couverture de bande de ceinture (1500) chevauche au moins une partie de l'un du premier bord extrême de stratifié de bande de ceinture, du second bord extrême de stratifié de bande de ceinture, du premier bord latéral de stratifié de bande de ceinture, et du second bord latéral de stratifié de bande de ceinture ;
dans lequel la couverture de bande de ceinture (1500) est jointe à une partie du châssis (52) et/ou à une partie du stratifié de bande de ceinture (1400) par une première liaison formant une partie d'un premier motif de liaison (2002) ;
dans lequel la couverture de bande de ceinture (1500) est jointe à une partie du châssis (52) et/ou à une partie du stratifié de bande de ceinture (1400) par une deuxième liaison formant une partie d'un deuxième motif de liaison (2004), et dans lequel le deuxième motif de liaison (2004) est différent du premier motif de liaison (2002) ; et
dans lequel une partie du châssis (52) à l'extérieur de la couverture de bande de ceinture (1500) comprend une troisième liaison formant une partie d'un troisième motif de liaison (2006), et dans lequel le troisième motif de liaison (2006) est sensiblement similaire à l'un du premier motif de liaison (2002) et du deuxième motif (2004).

2. Article absorbant selon la revendication 1, dans lequel la couverture de bande de ceinture (1500) chevauche complètement une surface entière du stratifié de bande de ceinture (1400).

3. Article absorbant selon la revendication 1, dans lequel la couverture de bande de ceinture (1500) est jointe à une partie de la feuille de dessus (26) ou à une partie de la couverture externe (40) par la première liaison formant une partie du premier motif de liaison (2002).

4. Article absorbant selon l'une quelconque des revendications précédentes, dans lequel une partie du second bord extrême de couverture de bande de ceinture n'est pas fixée au châssis (52) au moins dans une zone chevauchant l'axe longitudinal central (50) du châssis (52).

5. Article absorbant selon l'une quelconque des revendications précédentes, dans lequel la couverture de bande de ceinture (1500) définit une première pluralité d'ouvertures.

6. Article absorbant selon la revendication 5, dans lequel la feuille de dessus (26) et/ou la couverture externe (40) défini(ssen)t une seconde pluralité d'ouvertures, et dans lequel un premier motif formé par la première pluralité d'ouvertures se coordonne avec un second motif formé par la seconde pluralité d'ouvertures.

7. Article absorbant selon l'une quelconque des revendications précédentes, dans lequel le premier bord extrême de couverture de bande de ceinture coïncide avec le bord de ceinture avant (18) ou le bord de ceinture arrière (20).

8. Article absorbant selon l'une quelconque des revendications précédentes, comprenant une paire de pattes jointes à, et s'étendant vers l'extérieur de, la région de ceinture avant (12) et/ou la région de ceinture arrière (16), dans lequel une partie de la couverture de bande de ceinture (1500) est jointe à chaque patte de la paire de pattes.
